# EUROPEAN PATENT APPLICATION

(11) **EP 4 400 105 A1**
(43) Date of publication of application: **17.07.2024**
(21) Application number: 22867361.2
(22) Date of filing: 07.09.2022
(51) Int. Cl.: A61K 31/437, A61K 31/438, A61K 31/444, A61K 31/501, A61K 38/16, A61K 45/00, A61P 3/04, A61P 3/06, A61P 3/10, A61P 9/10, A61P 43/00

(54) **MEDICINE FOR PREVENTION AND TREATMENT OF DISEASES LINKED TO ANTI-OBESITY ACTIVITY**

(30) Priority: 08.09.2021 JP 2021146025
(71) Applicant: Shionogi & Co., Ltd, Osaka-shi, Osaka 541-0045 (JP)
(72) Inventor: TAKEMOTO Kosuke, Osaka-shi, Osaka 541-0045 (JP); KUSUMOTO Keisuke, Osaka-shi, Osaka 541-0045 (JP)
(74) Representative: Vossius & Partner Patentanwälte Rechtsanwälte mbB
(86) International application number: PCT/JP2022/033492
(87) International publication number: WO 2023/038039

(57) **Abstract**

A pharmaceutical preparation characterized by combination of (A) and (B) is useful for the prevention and treatment of a disease related to anti-obesity effect, wherein
(A) is a compound represented by formula: 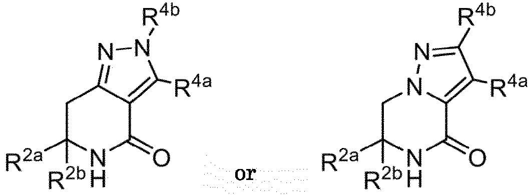 or a pharmaceutically acceptable salt thereof, and
(B) is at least one agent selected from the group consisting of agents having anti-obesity action, agents for controlling blood glucose level, agents for controlling cholesterol and/or triglyceride in blood, and agents for controlling blood pressure.

## Description

### [TECHNICAL FIELD]

The present invention relates to a pharmaceutical preparation for the prevention and treatment of a disease related to anti-obesity effect. More specifically, the present invention relates to a pharmaceutical preparation for the prevention and treatment of a disease related to anti-obesity effect, the therapeutic pharmaceutical preparation being characterized by combination of a compound having a monoacylglycerol acyltransferase 2 (hereinafter also referred to as MGAT2) inhibitory activity or a pharmaceutically acceptable salt thereof, with at least one agent selected from the group consisting of agents having anti-obesity effect, agents for controlling blood glucose level, agents for controlling cholesterol and/or triglyceride in blood, and agents for controlling blood pressure.

### [BACKGROUND ART]

Obesity is defined as a state in which an excessively high amount of body fat or adipose tissue in relation to lean body mass is accumulated, and is recognized as a major risk factor for health problems. Body mass index (BMI) is a simple index of weight-for-height that is commonly used in classifying overweight and obesity in adult (age 15 and over) populations and individuals. It is defined as the weight in kilograms divided by the square of the height in meters (kg/m²). World Health Organization defines "overweight" as a BMI of 25 kg/m² or greater and "obesity" as a BMI of 30 kg/m² or greater. On the other hand, Japan Society for the Study of Obesity defines "obesity" as a BMI of 25 kg/m² or greater. This is because the number of obesity-related diseases including diabetes and dislipidemia increases in accordance with BMI, and the mean number of obesity-related diseases is 1.0 or greater at a BMI of 25 kg/m². World Health Organization reported in the research in 2005 that about 1.6 billion people were classified as overweight, and at least 400 million people were classified as obesity, around the world. Obesity is mainly caused by taking in more calories than using up in physical activity and daily life. The number of obese people has been increasing by taking in more food including high fat and/or sugar, and it is estimated that 700 million people or more would be diagnosed as obesity around the world in 2015. Diet therapy, exercise therapy, drug therapy, and so on are performed for treatment of obesity. In the drug therapy, drugs including orlistat, mazindol, and sibutramine are used. However, they are not satisfactory in both aspects of efficacy and side effects.

Known as an agent for preventing or treating obesity is an agent containing a compound having appetite suppressing effects (selective serotonin reuptake inhibitors such as fenfluramine and fluoxetine; Mazindol, etc.), an agent containing a compound having nutrient digestion absorption suppressing effects, etc. Examples of the compound having nutrient digestion absorption suppressing effects include a compound having saccharide absorption suppressing effects (a-glucosidase inhibitors such as Acarbose and Voglibose; SGLT-2 inhibitors such as dapagliflozin, remogliflozin, and KGT-1075, and the like), and a compound having fat absorption suppressing effects. Examples of the compound having fat absorption suppressing effects include lipase inhibitors (compounds having gastric lipase inhibitory effects; compounds having pancreatic lipase inhibitory effects, such as orlistat, lipstatin, panclicin, and cetilistat, and the like), and bile acid adsorption resins such as cholestyramine and cholestyramide.

In addition, compounds acting on various mechanisms suggested to be associated with obesity have been researched and developed as candidates for anti-obesity drugs. Compounds considered candidates for anti-obesity drugs include 5HT transporter inhibitors, NE transporter inhibitors, CB-1 antagonists/inverse agonists, ghrelin antagonists, H3 antagonists/inverse agonists, MCH R1 antagonists, MCH R2 agonists/antagonists, NPY Y1 receptor antagonists, NPY Y2 receptor agonists, NPY Y4 receptor agonists, NPY Y5 receptor antagonists, mGluR5 antagonists, leptin, leptin agonists, leptin derivatives, opioid antagonists, orexin antagonists, BRS3 agonists, CCK-A agonists, CNTF, CNTF agonists, CNTF derivatives, GHS agonists, 5HT2C agonists, Mc4r agonists, monoamine reuptake inhibitors, GLP-1 agonists, UCP-1, 2 and 3 activators, β3 agonists, thyroid hormone β agonists, PDE inhibitors, FAS inhibitors, DGAT1 inhibitors, DGAT2 inhibitors, ACC2 inhibitors, glucocorticoid antagonists, acyl-estrogens, fatty acid transporter inhibitors, dicarboxylic acid transporter inhibitors, and the like.

Patent Documents 1 to 3 disclose compounds that are MGAT2 inhibitors, wherein the compounds is represented by formula: 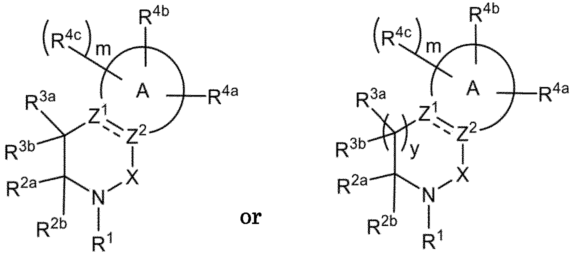 Other anti-obesity drugs are disclosed as agents that can be used with the above-described compound.

Patent Document 4 discloses compounds that are MGAT2 inhibitors, wherein the compounds is represented by formula: 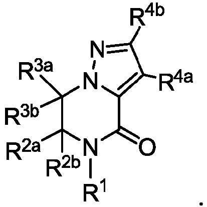 Other anti-obesity drugs are disclosed as agents that can be used with the above-described compound.

Patent Documents 5 and 6 disclose GLP-1 analogs. Patent Documents 7 to 24 and 27 to 50 disclose non-peptide small molecule GLP-1 agonists.

Patent Documents 25 to 26 disclose compounds that are ACC2 inhibitors, wherein the compounds is represented by formula: 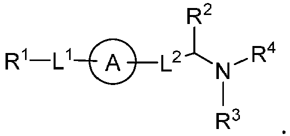 There is no description about agents that can be used with the above-described compounds.

Patent Document 51 discloses compounds that are NPY Y5 receptor antagonists, wherein the compounds is represented by formula:

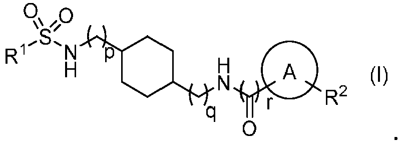

### [PRIOR ART REFERENCES]

### [Patent Documents]

[Patent Document 1] WO 2019013311 A
[Patent Document 2] WO 2019013312 A
[Patent Document 3] JP 2020-02515 A
[Patent Document 4] WO 2020145369 A
[Patent Document 5] WO 1998/08871 A
[Patent Document 6] WO 2006097537 A
[Patent Document 7] WO 2018109607 A
[Patent Document 8] WO 2019239319 A
[Patent Document 9] WO 2019239371 A
[Patent Document 10] WO 2021081207 A
[Patent Document 11] US Patent Application Publication 2011/0160198
[Patent Document 12] WO 2021096284 A
[Patent Document 13] WO 2021096304 A
[Patent Document 14] WO 2018056453 A
[Patent Document 15] WO 2020263695 A
[Patent Document 16] JP 2020-200308 A
[Patent Document 17] JP 2020-511411 A
[Patent Document 18] Chinese Patent Application Publication CN 113493447A
[Patent Document 19] WO 2021155841 A
[Patent Document 20] WO 2021242817 A
[Patent Document 21] WO 2020103815 A
[Patent Document 22] WO 2020207474 A
[Patent Document 23] WO 2021018023 A
[Patent Document 24] WO 2019103060 A
[Patent Document 25] WO 2015056782 A
[Patent Document 26] WO 2016159082 A
[Patent Document 27] WO 2021154796 A
[Patent Document 28] WO 2022031994 A
[Patent Document 29] WO 2021112538 A
[Patent Document 30] WO 2021160127 A
[Patent Document 31] WO 2021187886 A
[Patent Document 32] WO 2021197464 A
[Patent Document 33] WO 2021219019 A
[Patent Document 34] WO 2021244645 A
[Patent Document 35] WO 2021249492 A
[Patent Document 36] WO 2021259309 A
[Patent Document 37] WO 2021254470 A
[Patent Document 38] WO 2022028572 A
[Patent Document 39] WO 2022040600 A
[Patent Document 40] WO 2022042691 A
[Patent Document 41] WO 2022078380 A
[Patent Document 42] WO 2022078152 A
[Patent Document 43] WO 2022078407 A
[Patent Document 44] WO 2022109182 A
[Patent Document 45] WO 2022111624 A
[Patent Document 46] WO 2022111693 A
[Patent Document 47] Chinese Patent Application Publication CN 113480534A
[Patent Document 48] Chinese Patent Application Publication CN 113773310A
[Patent Document 49] Chinese Patent Application Publication CN 113816948A
[Patent Document 50] Chinese Patent Application Publication CN 113801136A
[Patent Document 51] WO 2012147764 A

### [SUMMARY OF THE INVENTION]

### [PROBLEMS TO BE SOLVED BY THE INVENTION]

It is an object of the present invention to provide a pharmaceutical preparation useful for the prevention and/or treatment of obesity or obesity-related diseases by using agents with different mechanisms in combination. In particular, it is an object of the present invention to provide a pharmaceutical preparation for the prevention and/or treatment of at least one selected from obesity, metabolic syndrome, hyperlipidemia, hypertriglyceridemia, hyper-VLDL-triglyceridemia, hyperfattyacidemia, diabetes mellitus, and arteriosclerosis.

### [MEANS FOR SOLVING THE PROBLEM]

The present invention provides aspects given below.
[1] A pharmaceutical preparation characterized in that the pharmaceutical preparation is characterized by combination of (A) and (B),
   wherein
   (A) is a compound represented by formula: 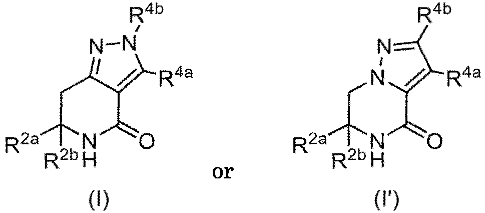 , or a pharmaceutically acceptable salt thereof,
      wherein
      R^{2a} and R^{2b} are taken together with an adjacent carbon atom to form a ring B, the ring B being represented by formula: 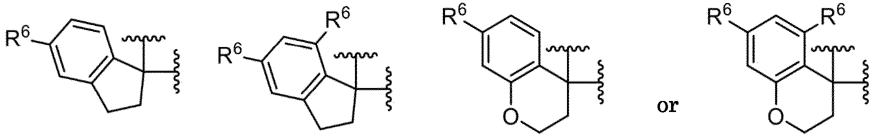 wherein R⁶s are each independently halogen, haloalkyl, alkyloxy, haloalkyloxy, or cyclopropanyl,
      R^{4a} is represented by formula: wherein
         L³ is alkylene, and
         R⁷ is alkylsulfamoyl, and
      R^{4b} is alkyl optionally substituted with substituent group α, a phenyl group optionally substituted with substituent group 6, or 5- to 6-membered aromatic heterocyclyl optionally substituted with substituent group β,
         wherein
         the substituent group α is halogen, haloalkyloxy, and cyclopropanyl, and
         the substituent group β is halogen, cyano, alkyl, haloalkyl, and cyclopropanyl, and
   (B) is at least one agent selected from the group consisting of agents having anti-obesity effect, agents for controlling blood glucose level, agents for controlling cholesterol and/or triglyceride in blood, and agents for controlling blood pressure.
[2] The pharmaceutical preparation according to [1],
   wherein
   R⁶s are each independently halogen, haloalkyl, or haloalkyloxy,
   R^{4a} is represented by formula: 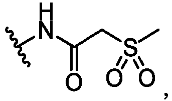 and
   R^{4b} is haloalkyloxyalkyl, a phenyl group optionally substituted with halogen, or 5- to 6-membered aromatic heterocyclyl optionally substituted with substituent group 6,
   wherein the substituent group B is halogen and alkyl.
[3] The pharmaceutical preparation according to [1] or [2],
   wherein (A) is a compound represented by formula: 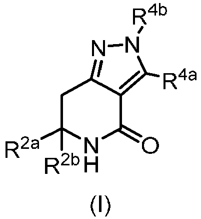 , or a pharmaceutically acceptable salt thereof,
      wherein
   R^{2a} and R^{2b} are taken together with an adjacent carbon atom to form a ring B, the ring B being represented by formula: 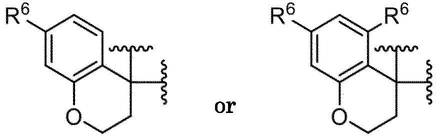 and the other symbols are as described in [1] or [2].
[4] The pharmaceutical preparation according to [1] or [2],
   wherein (A) is a compound represented by formula: 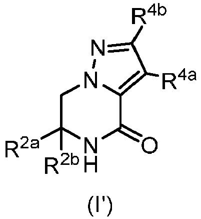 , or a pharmaceutically acceptable salt thereof,
   wherein the symbols are as described in [1] or [2].
[5] The pharmaceutical preparation according to [1],
   wherein (A) is a compound selected from the group consisting of compounds I-8, I-23, I-34, I-190, I-212, I-236, I-253, I-275, I-276, II-93, II-103, II-121, II-151, II-168, II-174, II-203, II-225, II-233, II-295, I'-6, I'-20, I'-33, I'-46, I'-58, I'-66, I'-79, I'-123, I'-124, I'-126, I'-130, I'-133, I'-136, I'-137, I'-139, I'-141, I'-142, I'-143, I'-145, I'-147, I'-151, and I'-153, or a pharmaceutically acceptable salt thereof.
[6] The pharmaceutical preparation according to any one of [1], [2], and [5],
   wherein (A) is a compound selected from the group consisting of compounds I-253, I-275, II-103, II-121, II-203, II-225, and II-233, or a pharmaceutically acceptable salt thereof.
[7] The pharmaceutical preparation according to any one of [1], [2], [4], and [5],
   wherein (A) is a compound selected from the group consisting of compounds I'-6, I'-66, I'-124, I'-137, I'-142, I'-147, and I'-151, or a pharmaceutically acceptable salt thereof.
[8] The pharmaceutical preparation according to any one of [1] to [7],
   wherein (B) is at least one selected from orlistat, cetilistat, phentermine, mazindol, benzphetamine, amfepramone, methamphetamine, phentermine hydrochloride/topiramate, naltrexone hydrochloride/bupropion hydrochloride, liraglutide, semaglutide, setmelanotide, metreleptin, topiramate, naltrexone, bupropion, acarbose, voglibose, miglitol, ipragliflozin, dapagliflozin, remogliflozin, KGT-1075, luseogliflozin, tofogliflozin, canagliflozin, empagliflozin, ertugliflozin, bexagliflozin, enavogliflozin henagliflozin, janagliflozin, sotagliflozin, insulin aspart, insulin lispro, insulin glulisine, biosynthetic human neutral insulin, human insulin, biosynthetic human isophene insulin, human isophene insulin, intermediate insulin lispro, insulin detemir, insulin glargine, insulin degludec, glibenclamide, gliclazide, glimepiride, glipizide, gliquidone, nateglinide, mitiglinide calcium hydrate, repaglinide, metformin hydrochloride, buformin hydrochloride, pioglitazone hydrochloride, rosiglitazone, lobeglitazone, sitagliptin phosphate, vildagliptin, alogliptin benzoate, linagliptin, teneligliptin hydrobromide, anagliptin, saxagliptin, trelagliptin succinate, omarigliptin, gemigliptin, evogliptin, lixisenatide, exenatide, dulaglutide, tirzepatide, imegurimin, sitagliptin phosphate/ipragliflozin, pioglitazone hydrochloride/metformin, pioglitazone hydrochloride/glimepiride, teneligliptin hydrobromide/canagliflozin, alogliptin benzoate/pioglitazone hydrochloride, alogliptin benzoate/metformin hydrochloride, vildagliptin/metformin hydrochloride, mitiglinide calcium/voglibose, pravastatin, simvastatin, fluvastatin, atorvastatin, pitavastatin, rosuvastatin, lovastatin, clinofibrate, clofibrate, bezafibrate, fenofibrate, ciprofibrate, pemafibrate, gemfibrozil, colestimide, colestyramine, colesevelam hydrochloride, colestipol, ezetimibe, blobcall, nicomol, tocopherol nicotinic acid ester, niceritrol, ethyl icosapentate, ω -3 fatty acid ethyl, evolocumab, alirocumab, nifedipine, amlodipine, efonidipine, cilnidipine, nicardipine, nisoldipine, nitrendipine, nilvadipine, barnidipine, felodipine, benidipine, manidipine, azelnidipine, alanidipine, diltiazem, trichlormethiazide, benzylhydrochlorothiazide, hydrochlorothiazide, macrone, indapamide, tripamide, mefluside, furosemide, triamterene, spironolactone, eplerenone, tolvaptan, torasemide, hydrochlorothiazide, bumetanide, chlorthalidone, isosorbide, metolazone,losartan, candesartan, valsartan, telmisartan, olmesartan, irbesartan, azilsartan, captopril, enalapril, alacepril, delapril, cilazapril, lisinopril, benazepril, imidapril, temocapril, quinapril, trandolapril, perindopril erbumine, urapidil, terazosin, prazosin, doxazosin, bunazosin, atenolol, bisoprolol, metoprolol, acebutolol, celiprolol, propranolol, nadolol, nipradilol, carteolol, pindolol, nebivolol, carvedilol, labetalol, sotalol, landiolol, arotinolol, amosulalol, arotinolol, carvedilol, labetalol, bevantolol, clonidine, guanabenz, methyldopa, reserpine, hydralazine, nitroprusside, aliskiren, kallidinogenase, alprostadil alfadex, dihydroergotoxine, doxazosin, urapidil, hydralazine, prazosin, moxonidine, guanfacine, rilmenidine, amlodipine/atrovastatin, losartan/hydrochlorothiazide, valsartan/hydrochlorothiazide, candesartan/hydrochlorothiazide, telmisartan/hydrochlorothiazide, irbesartan/trichlorothiazide, valsartan/amlodipine, olmesartan/azelnidipine, candesartan/amlodipine, telmisartan/amlodipine, irbesartan/amlodipine, valsartan/cilnidipine, azilsartan/amlodipine, tirzepatide, SCO-094, HM12525A, BI-456906, DD01, NN-9423, LY-3437943, danuglipron, PF07081532, LY-3502970, RGT-075, efpeglenatide, exenatide, Noiiglutide, GMA105, HM-15136, noliglycopeptide, LV-101, PYY-1562, PYY-1875, cotadutide/AM833, cotadutide, LY3305677, pegapamodutide, NGM395, YH34160, CT-388, CT-868, SCO-267, SCO-792, diazoxide, tesofensine, namodenoson, ERX1000, AMG133, ASC41, Xlal, HDV Biotin, EMP16, metoprolol/tesofensine, RZL-012, CB4211, BI1356225, AMG171, NO-13065, bardoxolone methyl, HSG4112, YHC2129, YHC2134, KTX-0200, obeticholic acid, GS-9674, LJN452, EDP-305, EYP-001, resmetirom, VK-2809, cenicriviroc, saroglitazar, lanifibranor, selonsertib, PF-06835919, pegbelfermin, efrugxifermin, aldafermin, aramcol, MK-3655, MSDC-0602K, belapectin, GS-0976, PF-05221304, ervogastat, ION-224, AXA-1125, HU-6, MET-409, MET-642, TERN-101, TERN-501, LPCN-1144, denifanstat, fruxifermin, leronlimab, pegozafermin, rencofilstat, retatrutide, tipelukast, S-237648, and S-723595.
[8'] The pharmaceutical preparation according to any one of [1] to [7],
   wherein (B) is at least one selected from orlistat, cetilistat, phentermine, mazindol, benzphetamine, amfepramone, methamphetamine, phentermine hydrochloride/topiramate, naltrexone hydrochloride/bupropion hydrochloride, liraglutide, semaglutide, setmelanotide, metreleptin, topiramate, naltrexone, bupropion, acarbose, voglibose, miglitol, ipragliflozin, dapagliflozin, luseogliflozin, tofogliflozin, canagliflozin, empagliflozin, ertugliflozin, bexagliflozin, enavogliflozin henagliflozin, janagliflozin, sotagliflozin, insulin aspart, insulin lispro, insulin glulisine, biosynthetic human neutral insulin, human insulin, biosynthetic human isophene insulin, human isophene insulin, intermediate insulin lispro, insulin detemir, insulin glargine, insulin degludec, glibenclamide, gliclazide, glimepiride, glipizide, gliquidone, nateglinide, mitiglinide calcium hydrate, repaglinide, metformin hydrochloride, buformin hydrochloride, pioglitazone hydrochloride, rosiglitazone, lobeglitazone, sitagliptin phosphate, vildagliptin, alogliptin benzoate, linagliptin, teneligliptin hydrobromide, anagliptin, saxagliptin, trelagliptin succinate, omarigliptin, gemigliptin, evogliptin, lixisenatide, exenatide, dulaglutide, tirzepatide, imegurimin, sitagliptin phosphate/ipragliflozin, pioglitazone hydrochloride/metformin, pioglitazone hydrochloride/glimepiride, teneligliptin hydrobromide/canagliflozin, alogliptin benzoate/pioglitazone hydrochloride, alogliptin benzoate/metformin hydrochloride, vildagliptin/metformin hydrochloride, mitiglinide calcium/voglibose, pravastatin, simvastatin, fluvastatin, atorvastatin, pitavastatin, rosuvastatin, lovastatin, clinofibrate, clofibrate, bezafibrate, fenofibrate, ciprofibrate, pemafibrate, gemfibrozil, colestimide, colestyramine, colesevelam hydrochloride, colestipol, ezetimibe, blobcall, nicomol, tocopherol nicotinic acid ester, niceritrol, ethyl icosapentate, ω-3 fatty acid ethyl, evolocumab, alirocumab, nifedipine, amlodipine, efonidipine, cilnidipine, nicardipine, nisoldipine, nitrendipine, nilvadipine, barnidipine, felodipine, benidipine, manidipine, azelnidipine, alanidipine, diltiazem, trichlormethiazide, benzylhydrochlorothiazide, hydrochlorothiazide, macrone, indapamide, tripamide, mefluside, furosemide, triamterene, spironolactone, eplerenone, tolvaptan, torasemide, hydrochlorothiazide, bumetanide, chlortalidone, isosorbide, metolazone,losartan, candesartan, valsartan, telmisartan, olmesartan, irbesartan, azilsartan, captopril, enalapril, alacepril, delapril, cilazapril, lisinopril, benazepril, imidapril, temocapril, quinapril, trandolapril, perindopril erbumine, urapidil, terazosin, prazosin, doxazosin, bunazosin, atenolol, bisoprolol, metoprolol, acebutolol, celiprolol, propranolol, nadolol, nipradilol, carteolol, pindolol, nebivolol, carvedilol, labetalol, sotalol, landiolol, arotinolol, amosulalol, arotinolol, carvedilol, labetalol, bevantolol, clonidine, guanabenz, methyldopa, reserpine, hydralazine, nitroprusside, aliskiren, kallidinogenase, alprostadil alfadex, dihydroergotoxine, doxazosin, urapidil, hydralazine, prazosin, moxonidine, guanfacine, rilmenidine, amlodipine/atrovastatin, losartan/hydrochlorothiazide, valsartan/hydrochlorothiazide, candesartan/hydrochlorothiazide, telmisartan/hydrochlorothiazide, irbesartan/trichlorothiazide, valsartan/amlodipine, olmesartan/azelnidipine, candesartan/amlodipine, telmisartan/amlodipine, irbesartan/amlodipine, valsartan/cilnidipine, azilsartan/amlodipine, tirzepatide, SCO-094, HM12525A, BI-456906, DD01, NN-9423, LY-3437943, danuglipron, efpeglenatide, exenatide, noiiglutide, GMA105, HM-15136, noliglycopeptide, LV-101, PYY-1562, PYY-1875, cotadutide/AM833, cotadutide, LY3305677, pegapamodutide, NGM395, YH34160, CT-388, CT-868, SCO-267, SCO-792, diazoxide, tesofensine, namodenoson, ERX1000, AMG133, ASC41, Xlal, HDV Biotin, EMP16, metoprolol/tesofensine, RZL-012, CB4211, BI1356225, AMG171, NO-13065, bardoxolone methyl, HSG4112, YHC2129, YHC2134, KTX-0200, obeticholic acid, GS-9674, LJN452, EDP-305, resmetirom, VK-2809, cenicriviroc, saroglitazar, lanifibranor, selonsertib, PF-06835919, pegbelfermin, efrugxifermin, aldafermin, aramcol, MK-3655, MSDC-0602K, belapectin, GS-0976, PF-05221304, and S-723595.
[9] The pharmaceutical preparation according to any one of [1] to [7], wherein (B) is at least one selected from GLP-1 agonists, GLP-1 analogs, and ACC2 inhibitors.
[9'] The pharmaceutical preparation according to any one of [1] to [7], wherein (B) is at least one selected from GLP-1 agonists, GLP-1 analogs, NPY Y5 receptor antagonists, and ACC2 inhibitors.
[10] The pharmaceutical preparation according to [9], wherein (B) is at least one selected from liraglutide, semaglutide, tirzepatide, BI-456906, danuglipron, S-723595, and pharmaceutically acceptable salts thereof.
[10'] The pharmaceutical preparation according to [9'], wherein (B) is at least one selected from liraglutide, semaglutide, tirzepatide, BI-456906, danuglipron, PF07081532, LY-3502970, RGT-075, S-237648, S-723595, and pharmaceutically acceptable salts thereof.
[11] The pharmaceutical preparation according to any one of [1] to [8], [8'], [9], [9'], [10], and [10'], wherein (A) and (B) are administered in combination.
[12] The pharmaceutical preparation according to any one of [1] to [8], [8'], [9], [9'], [10], and [10'], wherein the pharmaceutical preparation is a combination drug.
[13] The pharmaceutical preparation according to any one of [1] to [8], [8'], [9], [9'], [10], [10'], [11], and [12] for the prevention and/or treatment of at least one selected from obesity, metabolic syndrome, hyperlipidemia, hypertriglyceridemia, hyper-VLDL-triglyceridemia, hyperfattyacidemia, diabetes mellitus, and arteriosclerosis.
[14] An effect enhancer for (B) according to [1] comprising (A) according to [1].
[15] An effect enhancer for (A) according to [1] comprising (B) according to [1].
[16] A pharmaceutical preparation for administration in combination with (B) according to [1] comprising (A) according to [1] as an active ingredient.
[17] A pharmaceutical preparation for administration in combination with (A) according to [1] comprising (B) according to [1] as an active ingredient.

### [EFFECT OF THE INVENTION]

The pharmaceutical preparation of the present invention is useful for the prevention and/or treatment of obesity or obesity-related diseases. In particular, it is useful for the prevention and/or treatment of at least one selected from obesity, metabolic syndrome, hyperlipidemia, hypertriglyceridemia, hyper-VLDL-triglyceridemia, hyperfattyacidemia, diabetes mellitus, and arteriosclerosis.

### [BRIEF DESCRIPTION OF DRAWINGS]

Fig. 1 shows the results of daily weight change measured according to Test Example 2 using Compound II-121 as the compound (A) and liraglutide as the compound (B).
Fig. 2 shows the results of daily weight change measured according to Test Example 2 using Compound II-121 as the compound (A) and semaglutide as the compound (B).

### [MODE FOR CARRYING OUT THE INVENTION]

Hereinafter, the meaning of each term used in the present specification will be described. Unless particularly stated otherwise, each term is used in the same sense, either alone or in combination with other terms.

The term "consist of" means having only the constituent elements.

The term "comprise" means that elements are not limited to the constituent elements, and elements that are not described are not excluded.

Hereinafter, the present invention will be described while showing exemplary embodiments. Throughout the present specification, it should be understood that, unless particularly stated otherwise, an expression of a singular form also includes the concept of a plural form thereof. Therefore, it should be understood that, unless particularly stated otherwise, an article for a singular form (for example, in the case of English, "a", "an", "the", or the like) also includes the concept of a plural form thereof.

Furthermore, it should be understood that, unless particularly stated otherwise, the terms used in the present specification are used in the meanings normally used in the above-described art. Accordingly, unless otherwise defined, all terminologies and scientific and technical terms used in the present specification have the same meanings as commonly understood by those having ordinary skill in the art to which the present invention belongs. In a case of contradiction, priority is given to the present specification (including definitions).

"Halogen" encompasses a fluorine atom, a chlorine atom, a bromine atom, and an iodine atom. In particular, a fluorine atom and a chlorine atom are preferable.

"Alkyl" encompasses linear or branched hydrocarbon groups each having 1 to 15 carbon atoms, preferably 1 to 10 carbon atoms, more preferably 1 to 6 carbon atoms, and even more preferably 1 to 4 carbon atoms. Examples include methyl, ethyl, n-propyl, isopropyl, n-butyl, isobutyl, sec-butyl, tert-butyl, n-pentyl, isopentyl, neopentyl, n-hexyl, isohexyl, n-heptyl, isoheptyl, n-octyl, isooctyl, n-nonyl, and n-decyl.

Preferred embodiments of "alkyl" include methyl, ethyl, n-propyl, isopropyl, n-butyl, isobutyl, sec-butyl, tert-butyl, and n-pentyl. More preferred embodiments include methyl, ethyl, n-propyl, isopropyl, and tert-butyl.

"Alkylene" encompasses linear or branched divalent hydrocarbon groups each having 1 to 15 carbon atoms, preferably 1 to 10 carbon atoms, more preferably 1 to 6 carbon atoms, and even more preferably 1 to 4 carbon atoms. Examples include methylene, ethylene, trimethylene, propylene, tetramethylene, pentamethylene, hexamethylene.

An "aromatic heterocyclyl" means an aromatic cyclic group, which is monocyclic or polycyclic having two or more rings, having one or more, same or different heteroatom(s) selected optionally from O, S, and N.

An aromatic heterocyclyl having two or more rings also encompasses an aromatic heterocyclyl having a single ring or two or more rings, to which a ring in the "aromatic carbocyclyl" is fused, and the linking bond may be carried by any of the rings.

The aromatic heterocyclyl having a single ring is preferably a 5- to 8-membered ring, and more preferably a 5-membered or 6-membered ring. Examples of 5-membered aromatic heterocyclyl include pyrrolyl, imidazolyl, pyrazolyl, triazolyl, tetrazolyl, furyl, thienyl, isoxazolyl, oxazolyl, oxadiazolyl, isothiazolyl, thiazolyl, and thiadiazolyl. Examples of 6-membered aromatic heterocyclyl include pyridyl, pyridazinyl, pyrimidinyl, pyrazinyl, and triazinyl.

The aromatic heterocyclyl having two rings is preferably a 8- to 10-membered ring, and more preferably a 9-membered or 10-membered ring. Examples thereof include indolyl, isoindolyl, indazolyl, indolizinyl, quinolinyl, isoquinolinyl, cinnolinyl, phthalazinyl, quinazolinyl, naphthyridinyl, quinoxalinyl, purinyl, pteridinyl, benzimidazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, imidazopyridyl, triazolopyridyl, imidazothiazolyl, pyrazinopyridazinyl, oxazolopyridyl, and thiazolopyridyl.

The aromatic heterocyclyl having three or more rings is preferably a 13- to 15-membered group. Examples include carbazolyl, acridinyl, xanthenyl, phenothiazinyl, phenoxathiinyl, phenoxazinyl, and dibenzofuryl.

The expression "optionally substituted with substituent group α" herein means "optionally substituted with one or more groups selected from the substituent group α". The same is true in the substituent group B.

The present invention is a pharmaceutical preparation characterized by combination of (A) and (B),
wherein
(A) is a compound represented by formula: 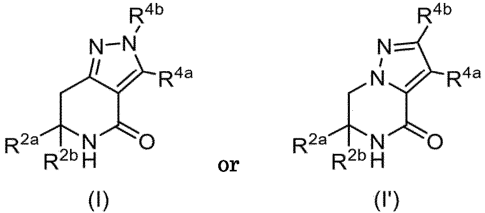 , or a pharmaceutically acceptable salt thereof,
   wherein
   R^{2a} and R^{2b} are taken together with an adjacent carbon atom to form a ring B, the ring B being represented by formula: 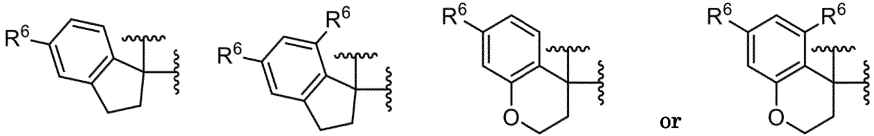 wherein R⁶s are each independently halogen, haloalkyl, alkyloxy, haloalkyloxy, or cyclopropanyl,
   R^{4a} is represented by formula: 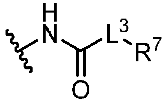 wherein
      L³ is alkylene, and
      R⁷ is alkylsulfamoyl; and
   R^{4b} is alkyl optionally substituted with substituent group α, a phenyl group optionally substituted with substituent group B, or 5- to 6-membered aromatic heterocyclyl optionally substituted with substituent group β,
      wherein the substituent group α is halogen, haloalkyloxy, and cyclopropanyl, and
      the substituent group β is halogen, cyano, alkyl, haloalkyl, and cyclopropanyl, and
(B) is at least one agent selected from the group consisting of agents having anti-obesity effect, agents for controlling blood glucose level, agents for controlling cholesterol and/or triglyceride in blood, and agents for controlling blood pressure.

The "at least one agent selected from the group consisting of agents having anti-obesity effect, agents for controlling blood glucose level, agents for controlling cholesterol and/or triglyceride in blood, and agents for controlling blood pressure" of (B) to be combined with (A) may be any agent, as long as it has such effects. However, the "at least one agent selected from the group consisting of agents having anti-obesity effect, agents for controlling blood glucose level, agents for controlling cholesterol and/or triglyceride in blood, and agents for controlling blood pressure" used in (B) is a compound different from the compound used as (A), or a pharmaceutically acceptable salt thereof.

The "at least one agent selected from the group consisting of agents having anti-obesity effect, agents for controlling blood glucose level, agents for controlling cholesterol and/or triglyceride in blood, and agents for controlling blood pressure" include, but is not limited to, a commercially available agent and an agent under development. Examples of the commercially available agent or the agent under development include:orlistat, cetilistat, phentermine, mazindol, benzphetamine, amfepramone, methamphetamine, phentermine hydrochloride/topiramate, naltrexone hydrochloride/bupropion hydrochloride, liraglutide, semaglutide, setmelanotide (RM-493), metreleptin, topiramate, naltrexone, bupropion, acarbose, voglibose, miglitol, ipragliflozin, dapagliflozin, remogliflozin, KGT-1075, luseogliflozin, tofogliflozin, canagliflozin, empagliflozin, ertugliflozin, bexagliflozin, enavogliflozin henagliflozin, janagliflozin, sotagliflozin, insulin aspart, insulin lispro, insulin glulisine, biosynthetic human neutral insulin, human insulin, biosynthetic human isophene insulin, human isophene insulin, intermediate insulin lispro, insulin detemir, insulin glargine, insulin degludec, glibenclamide, gliclazide, glimepiride, glipizide, gliquidone, nateglinide, mitiglinide calcium hydrate, repaglinide, metformin hydrochloride, buformin hydrochloride, pioglitazone hydrochloride, rosiglitazone, lobeglitazone, sitagliptin phosphate, vildagliptin, alogliptin benzoate, linagliptin, teneligliptin hydrobromide, anagliptin, saxagliptin, trelagliptin succinate, omarigliptin, gemigliptin, evogliptin, lixisenatide, exenatide, dulaglutide, tirzepatide, imegurimin, sitagliptin phosphate/ipragliflozin, pioglitazone hydrochloride/metformin, pioglitazone hydrochloride/glimepiride, teneligliptin hydrobromide/canagliflozin, alogliptin benzoate/pioglitazone hydrochloride, alogliptin benzoate/metformin hydrochloride, vildagliptin/metformin hydrochloride, mitiglinide calcium/voglibose, pravastatin, simvastatin, fluvastatin, atorvastatin, pitavastatin, rosuvastatin, lovastatin, clinofibrate, clofibrate, bezafibrate, fenofibrate, ciprofibrate, pemafibrate, gemfibrozil, colestimide, colestyramine, colesevelam hydrochloride, colestipol, ezetimibe, blobcall, nicomol, tocopherol nicotinic acid ester, niceritrol, ethyl icosapentate, ω-3 fatty acid ethyl, evolocumab, alirocumab, nifedipine, amlodipine, efonidipine, cilnidipine, nicardipine, nisoldipine, nitrendipine, nilvadipine, barnidipine, felodipine, benidipine, manidipine, azelnidipine, alanidipine, diltiazem, trichlormethiazide, benzylhydrochlorothiazide, hydrochlorothiazide, macrone, indapamide, tripamide, mefluside, furosemide, triamterene, spironolactone, eplerenone, tolvaptan, torasemide, hydrochlorothiazide, bumetanide, chlortalidone, isosorbide, metolazone,losartan, candesartan, valsartan, telmisartan, olmesartan, irbesartan, azilsartan, captopril, enalapril, alacepril, delapril, cilazapril, lisinopril, benazepril, imidapril, temocapril, quinapril, trandolapril, perindopril erbumine, urapidil, terazosin, prazosin, doxazosin, bunazosin, atenolol, bisoprolol, metoprolol, acebutolol, celiprolol, propranolol, nadolol, nipradilol, carteolol, pindolol, nebivolol, carvedilol, labetalol, sotalol, landiolol, arotinolol, amosulalol, arotinolol, carvedilol, labetalol, bevantolol, clonidine, guanabenz, methyldopa, reserpine, hydralazine, nitroprusside, aliskiren, kallidinogenase, alprostadil alfadex, dihydroergotoxine, doxazosin, urapidil, hydralazine, prazosin, moxonidine, guanfacine, rilmenidine, amlodipine/atrovastatin, losartan/hydrochlorothiazide, valsartan/hydrochlorothiazide, candesartan/hydrochlorothiazide, telmisartan/hydrochlorothiazide, irbesartan/trichlorothiazide, valsartan/amlodipine, olmesartan/azelnidipine, candesartan/amlodipine, telmisartan/amlodipine, irbesartan/amlodipine, valsartan/cilnidipine, azilsartan/amlodipine, tirzepatide, SCO-094, efinopegdutide (HM12525A), BI-456906, DD01, NN-9423, LY-3437943, danuglipron, PF07081532, LY-3502970, RGT-075, efpeglenatide, exenatide, noiiglutide, GMA105, HM-15136, noliglycopeptide (SHR-20004), carbetocin (LV-101), PYY-1562 (NNC0165-1562), PYY-1875 (NNC0165-1875), cotadutide/AM833 (NN9838, NNC-01740833), cotadutide (MEDI0382), LY3305677, pegapamodutide (LY2944876, OPK88003), NGM395, YH34160, CT-388, CT-868, SCO-267, SCO-792, diazoxide, tesofensine, namodenoson, ERX1000, AMG133, ASC41, Xlal, HDV Biotin, EMP16, metoprolol/tesofensine, RZL-012, CB4211, BI1356225, AMG171, NO-13065, bardoxolone methyl, HSG4112, YHC2129, YHC2134, KTX-0200, obeticholic acid, cilofexor (GS-9674), tropifexor (LJN452), EDP-305, EYP-001, resmetirom, VK-2809, cenicriviroc, saroglitazar, lanifibranor, selonsertib, PF-06835919, pegbelfermin, efrugxifermin, aldafermin, aramcol, MK-3655, MSDC-0602K, belapectin, firsocostat (GS-0976), PF-05221304, ervogastat, ION-224, AXA-1125, HU-6, MET-409, MET-642, TERN-101, TERN-501, LPCN-1144, denifanstat, fruxifermin, leronlimab, pegozafermin, rencofilstat, retatrutide, tipelukast, S-237648, and S-723595. Furthermore, when these agents do not form a salt, pharmaceutically acceptable salts thereof are also included, and when they form a salt, other pharmaceutically acceptable salts may be formed.

Preferable examples include orlistat, cetilistat, phentermine, mazindol, benzphetamine, amfepramone, methamphetamine, phentermine hydrochloride/topiramate, naltrexone hydrochloride/bupropion hydrochloride, liraglutide, semaglutide, setmelanotide (RM-493), metreleptin, topiramate, naltrexone, bupropion, tirzepatide, SCO-094, efinopegdutide (HM12525A), BI-456906, DD01, NN-9423, LY-3437943, danuglipron, PF07081532, LY-3502970, RGT-075, efpeglenatide, exenatide, noiiglutide, GMA105, HM-15136, noliglycopeptide (SHR-20004), carbetocin (LV-101), PYY-1562 (NNC0165-1562), PYY-1875 (NNC0165-1875), cotadutide/AM833 (NN9838, NNC-01740833), cotadutide (MEDI0382), LY3305677, pegapamodutide (LY2944876, OPK88003), NGM395, YH34160, CT-388, CT-868, SCO-267, SCO-792, diazoxide, tesofensine, namodenoson, ERX1000, AMG133, ASC41, Xlal, HDV Biotin, EMP16, metoprolol/tesofensine, RZL-012, CB4211, BI1356225, AMG171, NO-13065, bardoxolone methyl, HSG4112, YHC2129, YHC2134, KTX-0200, S-237648, and S-723595.

Further preferable examples include liraglutide, semaglutide, tirzepatide, BI-456906, danuglipron, PF07081532, LY-3502970, RGT-075, S-237648, and S-723595.

Preferable examples of the "at least one agent selected from the group consisting of agents having anti-obesity effect, agents for controlling blood glucose level, agents for controlling cholesterol and/or triglyceride in blood, and agents for controlling blood pressure" include a GLP-1 agonist, a GLP-1 analog, an NPY-Y5 receptor antagonist, and an ACC2 inhibitor.

The "GLP-1 agonist" may be anything as long as it has a GLP-1 agonist effect and falls under the category of the "at least one agent selected from the group consisting of agents having anti-obesity effect, agents for controlling blood glucose level, agents for controlling cholesterol and/or triglyceride in blood, and agents for controlling blood pressure".

Examples of the "GLP-1 agonist" include, but are not limited to, the compounds described below and salts thereof:
danuglipron (formerly PF06882961);
PF07081532;
LY-3502970;
RGT-075;

In addition, examples of the same include, but are not limited to, compounds and salts thereof described in the following patent documents:
WO 2018109607;
WO 2019239319;
WO 2019239371;
WO 2021081207;
WO 2021154796;
US 20110160198;
WO 2021096284;
WO 2021096304;
WO 2018056453;
WO 2020263695;
JP 2020200308A;
JP 2020511411A;
WO 2020103815;
WO 2020207474;
WO 2021018023;
WO 2021155841;
WO 2019103060;
WO 2022031994;
WO 2021112538;
WO 2021160127;
WO 2021187886;
WO 2021197464;
WO 2021219019;
WO 2021242817;
WO 2021244645;
WO 2021249492;
WO 2021259309;
WO 2021254470;
WO 2022028572;
WO 2022040600;
WO 2022042691;
WO 2022078380;
WO 2022078152;
WO 2022078407;
WO 2022109182;
WO 2022111624;
WO 2022111693;
Chinese Patent Application Publication CN 113493447A
Chinese Patent Application Publication CN 113480534A
Chinese Patent Application Publication CN 113773310A
Chinese Patent Application Publication CN 113816948A
Chinese Patent Application Publication CN 113801136A

Examples of the "GLP-1 analog" include, but are not limited to, the peptides described below:
liraglutide;
lixisenatide;
exenatide;
semaglutide;
dulaglutide;
efpeglenatide;
exenatide;
noiiglutide;
GMA105;
HM-15136;
noliglycopeptide (SHR-20004);
tirzepatide;
BI-456906

The "ACC2 inhibitor" may be anything as long as it has an ACC2 inhibitor effect and falls under the category of the "at least one agent selected from the group consisting of agents having anti-obesity effect, agents for controlling blood glucose level, agents for controlling cholesterol and/or triglyceride in blood, and agents for controlling blood pressure".

Examples of the "ACC2 inhibitor" include, but are not limited to, the compounds described below and pharmaceutically acceptable salts thereof:
S-723595; 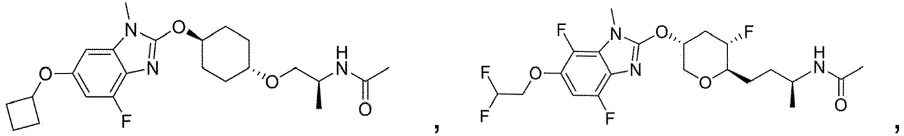 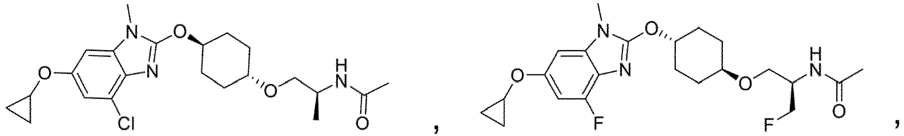 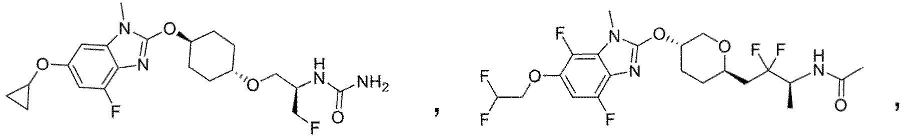 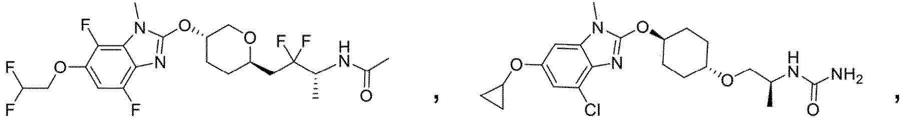 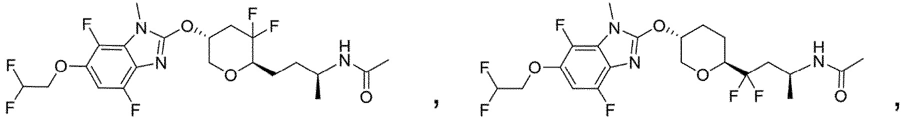 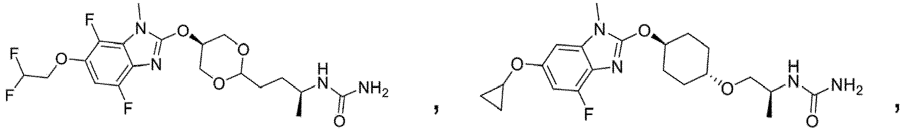 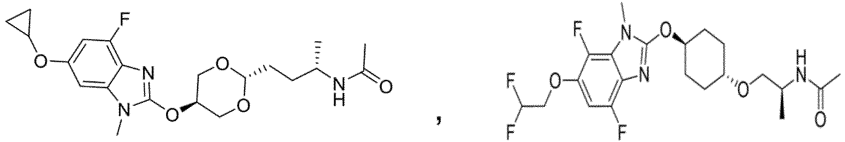 or

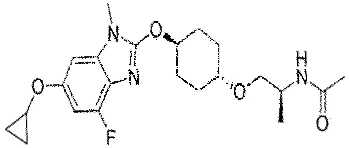

Examples of the ACC2 inhibitor preferably include the following compounds or pharmaceutically acceptable salts thereof. 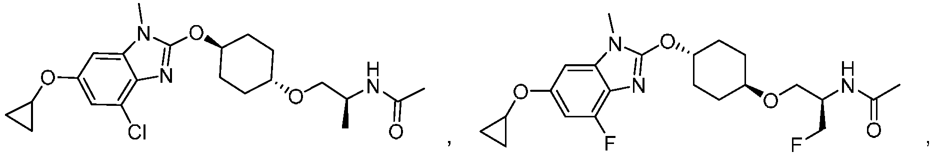 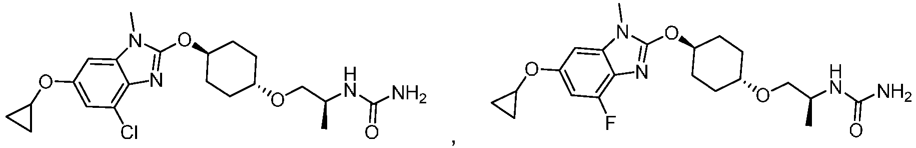 and

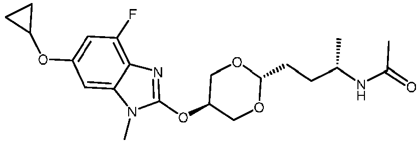

The "NPY Y5 receptor antagonist" may be anything as long as it has an NPY Y5 receptor antagonist effect and falls under the category of the "at least one agent selected from the group consisting of agents having anti-obesity effect, agents for controlling blood glucose level, agents for controlling cholesterol and/or triglyceride in blood, and agents for controlling blood pressure".

Examples of the "NPY Y5 receptor antagonist" include, but are not limited to, the compounds described below and pharmaceutically acceptable salts thereof:
S-237648; 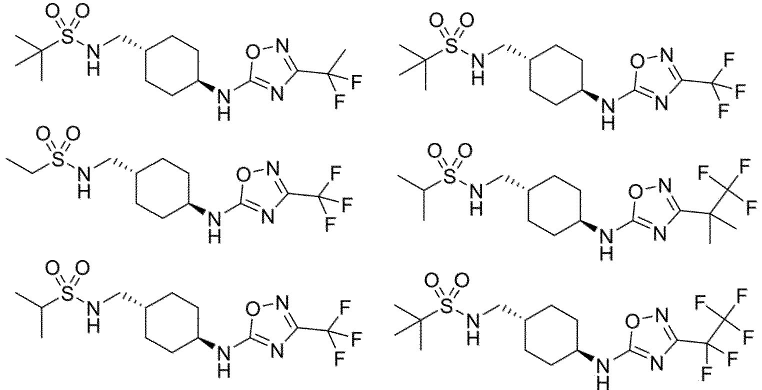 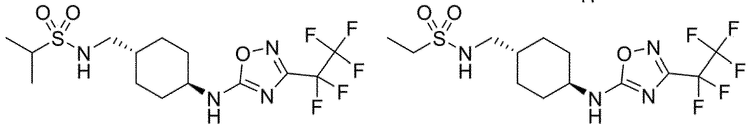 and

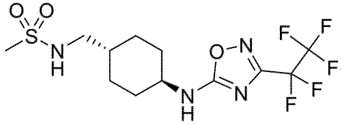

The "at least one agent selected from the group consisting of agents having anti-obesity effect, agents for controlling blood glucose level, agents for controlling cholesterol and/or triglyceride in blood, and agents for controlling blood pressure" may be one, two, or more agents, and does not have to be one agent.

The radiolabeled form of the compound to be used in the present invention can be prepared by the method well known in this technical field. For example, a tritium-labeled compound to be used in the present invention can be prepared by introducing tritium into a specific compound to be used in the present invention by catalytic dehalogenation reaction using tritium. This method encompasses reaction of a precursor which is a compound to be used in the present invention appropriately halogenated with tritium gas in the presence of an appropriate catalyst, for example, Pd/C, and in the presence or absence of a base. Regarding other appropriate methods for preparing tritium-labeled compounds, "Isotopes in the Physical and Biomedical Sciences, Vol. 1, Labeled Compounds (Part A), Chapter 6 (1987)" can be referred to. A ¹⁴C-labeled compound can be prepared by using a raw material having ¹⁴C carbon.

Examples of the pharmaceutically acceptable salt of the compound according to the present invention include salts of the compound according to the present invention with alkali metal (for example, lithium, sodium, potassium, etc.), alkaline earth metal (for example, calcium, barium, etc.), magnesium, transition metal (for example, zinc, iron, etc.), ammonia, organic base (for example, trimethylamine, triethylamine, dicyclohexylamine, ethanolamine, diethanolamine, triethanolamine, meglumine, ethylenediamine, pyridine, picoline, quinoline, etc.) and amino acid or salts of the compound according to the present invention with inorganic acid (for example, hydrochloric acid, sulfuric acid, nitric acid, carbonic acid, hydrobromic acid, phosphoric acid, hydroiodic acid, etc.), and organic acid (for example, formic acid, acetic acid, propionic acid, trifluoroacetic acid, citric acid, lactic acid, tartaric acid, oxalic acid, maleic acid, fumaric acid, succinic acid, mandelic acid, glutaric acid, malic acid, benzoic acid, phthalic acid, ascorbic acid, benzenesulfonic acid, p-toluenesulfonic acid, methanesulfonic acid, ethanesulfonic acid, trifluoroacetic acid, etc.). These salts can be formed according to methods that are conventionally carried out.

The compound of the present invention or its pharmaceutically acceptable salt may form a solvate (e.g., a hydrate), cocrystals and/or a crystal polymorph. The present invention also encompasses such various solvates, cocrystals and crystal polymorphs. "Solvates" may be those wherein any numbers of solvent molecules (e.g., water molecules or the like) are coordinated with the compounds of the present invention. When the compounds of the present invention or its pharmaceutically acceptable salts are allowed to stand in the atmosphere, the compounds may absorb water, resulting in attachment of adsorbed water or formation of hydrates. Recrystallization of the compounds of the present invention or its pharmaceutically acceptable salts may produce crystal polymorphs. "Cocrystal" means that the compound of the present invention or salt thereof and a counter molecule are present in the same crystal lattice, and a cocrystal with any number of counter molecules may be contained.

The compound to be used in the present invention or its pharmaceutically acceptable salt may form a prodrug. The present invention also encompasses such various prodrugs. A prodrug is a derivative of a compound of the present invention having a group that can be chemically or metabolically degraded, and is a compound which becomes a pharmaceutically active compound of the present invention in vivo as a result of solvolysis or under physiological conditions. Prodrugs encompasses compounds that are converted to the compounds represented by Formula (II) through enzymatic oxidation, reduction, hydrolysis, or the like under physiological conditions in vivo, compounds that are converted to the compounds represented by Formula (II) through hydrolysis by gastric acid etc., and the like. Methods for selecting and preparing suitable prodrug derivatives are described in, for example, "Design of Prodrugs, Elsevier, Amsterdam, 1985". A prodrug may have activity per se.

### (Method for producing compound to be used in present invention)

A general production method of the compound to be used in the present invention is exemplified below. The methods for extraction, purification and the like may be carried out by using the usual method for the experiments of organic chemistry.

The compounds to be used in the present invention can be synthesized by referring to the known methods in the art.

As the raw material compound, a commercially available compound, a compound described in the present specification, a compound described in a document cited in the present specification, and other known compounds can be utilized.

When it is desired to obtain a salt of the compound to be used in the present invention, when the compound to be used in the present invention is obtained in the form of a salt, the compound may be purified as it is, and when the compound to be used in the present invention is obtained in a free form, the compound may be dissolved or suspended in an appropriate organic solvent, and an acid or a base may be added to form a salt by an ordinary method.

The compound of (A) to be used in the present invention can be prepared, for example, by a method described in any of Patent Documents 1 to 4 and the like.

As the compound to be used in the present invention, given as (B), a commercially available compound may be used. Alternatively, the compound can be prepared, for example, by a method described in any of Patent Documents 5 to 51 and the like.

As one aspect, the pharmaceutical preparation of the present invention is a pharmaceutical preparation characterized by combination of (A) and (B),
wherein
(A) is a compound represented by formula: 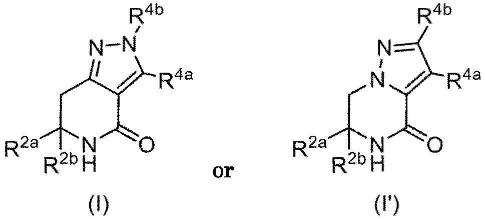 , or a pharmaceutically acceptable salt thereof,
   wherein
   R^{2a} and R^{2b} are taken together with an adjacent carbon atom to form a ring B, the ring B being represented by formula: 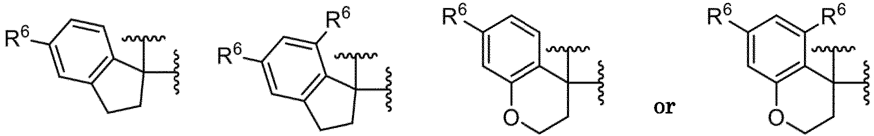 wherein R⁶s are each independently halogen, haloalkyl, alkyloxy, haloalkyloxy, or cyclopropanyl,
   R^{4a} is represented by formula: 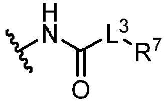 wherein
      L³ is alkylene, and
      R⁷ is alkylsulfamoyl; and
   R^{4b} is alkyl optionally substituted with substituent group α, a phenyl group optionally substituted with substituent group 6, or 5- to 6-membered aromatic heterocyclyl optionally substituted with substituent group β,
      wherein the substituent group α is halogen, haloalkyloxy, and cyclopropanyl, and
      the substituent group β is halogen, cyano, alkyl, haloalkyl, and cyclopropanyl, and
(B) is at least one agent selected from the group consisting of agents having anti-obesity effect, agents for controlling blood glucose level, agents for controlling cholesterol and/or triglyceride in blood, and agents for controlling blood pressure.

In another embodiment, the present invention provides a therapeutic pharmaceutical preparation for the prevention and/or treatment of obesity or obesity-related diseases, the preparation containing (A) and (B).

In another aspect, the present invention provides an effect enhancer in which (A) and (B) are combined.

The term "pharmaceutical preparation being characterized by combination" herein encompasses a pharmaceutical preparation containing each compound, an embodiment in which each compound is used as a combination drug, an embodiment in which each compound is used as a kit, an embodiment in which it is administered simultaneously, an embodiment in which it is administered at intervals, and an embodiment in which a certain medicament is used in combination with another medicament. Although there are also cases where the expression "combining (combined)" will be omitted, these cases have the same meaning. The pharmaceutical preparation is preferably a combination drug.

The compound given as (A) or a pharmaceutically acceptable salt thereof can be used in combination with (B) at least one agent selected from the group consisting of agents having anti-obesity effect, agents for controlling blood glucose level, agents for controlling cholesterol and/or triglyceride in blood, and agents for controlling blood pressure, and can enhance the effect of (B) at least one agent selected from the group consisting of agents having anti-obesity effect, agents for controlling blood glucose level, agents for controlling cholesterol and/or triglyceride in blood, and agents for controlling blood pressure.

In addition, (B) at least one agent selected from the group consisting of agents having anti-obesity effect, agents for controlling blood glucose level, agents for controlling cholesterol and/or triglyceride in blood, and agents for controlling blood pressure can be used in combination with the compound given as (A) or a pharmaceutically acceptable salt thereof, and can enhance the effect of the compound given as (A) or a pharmaceutically acceptable salt thereof.

### (Embodiment 1)

A pharmaceutical preparation characterized by combination of (A) and (B), wherein
(A) is a compound represented by formula: 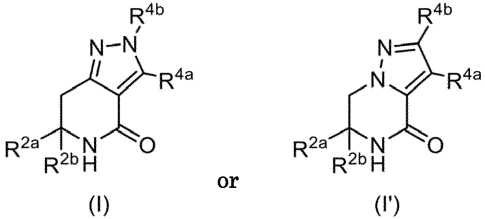 , or a pharmaceutically acceptable salt thereof,
   wherein
   R^{2a} and R^{2b} are taken together with an adjacent carbon atom to form a ring B, the ring B being represented by formula: 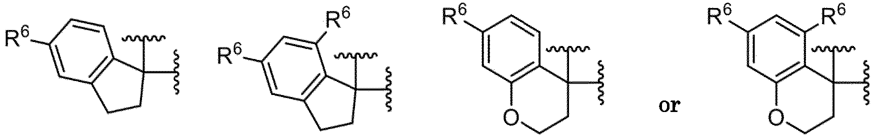 wherein R⁶s are each independently halogen, haloalkyl, alkyloxy, haloalkyloxy, or cyclopropanyl,
   R^{4a} is represented by formula: 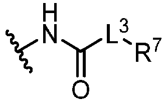 wherein
      L³ is alkylene, and
      R⁷ is alkylsulfamoyl; and
   R^{4b} is alkyl optionally substituted with substituent group α, a phenyl group optionally substituted with substituent group B, or 5- to 6-membered aromatic heterocyclyl optionally substituted with substituent group β,
      wherein
      the substituent group α is halogen, haloalkyloxy, and cyclopropanyl, and
      the substituent group β is halogen, cyano, alkyl, haloalkyl, and cyclopropanyl, and
(B) is at least one selected from GLP-1 agonists, GLP-1 analogs, NPY Y5 receptor antagonists, and ACC2 inhibitors.

### (Embodiment 2)

A pharmaceutical preparation characterized by combination of (A) and (B), wherein
(A) is a compound selected from the group consisting of I-8, I-23, I-34, I-190, I-212, I-236, I-253, I-275, I-276, II-93, II-103, II-121, II-151, II-168, II-174, II-203, II-225, II-233, II-295, I'-6, I'-20, I'-33, I'-46, I'-58, I'-66, I'-79, I'-123, I'-124, I'-126, I'-130, I'-133, I'-136, I'-137, I'-139, I'-141, I'-142, I'-143, I'-145, I'-147, I'-151, and I'-153, or a pharmaceutically acceptable salt thereof, and
(B) is at least one selected from orlistat, cetilistat, phentermine, mazindol, benzphetamine, amfepramone, methamphetamine, phentermine hydrochloride/topiramate, naltrexone hydrochloride/bupropion hydrochloride, liraglutide, semaglutide, setmelanotide (RM-493), metreleptin, topiramate, naltrexone, bupropion, tirzepatide, SCO-094, efinopegdutide (HM12525A), BI-456906, DD01, NN-9423, LY-3437943, danuglipron, PF07081532, LY-3502970, RGT-075, efpeglenatide, exenatide, noiiglutide, GMA105, HM-15136, noliglycopeptide (SHR-20004), carbetocin (LV-101), PYY-1562 (NNC0165-1562), PYY-1875 (NNC0165-1875), cotadutide/AM833 (NN9838, NNC-01740833), cotadutide (MEDI0382), LY3305677, pegapamodutide (LY2944876, OPK88003), NGM395, YH34160, CT-388, CT-868, SCO-267, SCO-792, diazoxide, tesofensine, namodenoson, ERX1000, AMG133, ASC41, Xlal, HDV Biotin, EMP16, metoprolol/tesofensine, RZL-012, CB4211, BI1356225, AMG171, NO-13065, bardoxolone methyl, HSG4112, YHC2129, YHC2134, KTX-0200, S-237648, and S-723595.

### (Embodiment 2')

A pharmaceutical preparation characterized by combination of (A) and (B), wherein
(A) is a compound selected from the group consisting of compounds I-8, I-23, I-34, I-190, I-212, I-236, I-253, I-275, I-276, II-93, II-103, II-121, II-151, II-168, II-174, II-203, II-225, II-233, II-295, I'-6, 1' 20, I'-33, 1' 46, I'-58, 1' 66, 1' 79, I'-123, I'-124, I'-126, I'-130, I'-133, I'-136, I'-137, I'-139, I'-141, I'-142, I'-143, I'-145, I'-147, I'-151, and I'-153, or a pharmaceutically acceptable salt thereof, and
(B) is at least one selected from orlistat, cetilistat, phentermine, mazindol, benzphetamine, amfepramone, methamphetamine, phentermine hydrochloride/topiramate, naltrexone hydrochloride/bupropion hydrochloride, liraglutide, semaglutide, setmelanotide (RM-493), metreleptin, topiramate, naltrexone, bupropion, tirzepatide, SCO-094, efinopegdutide (HM12525A), BI-456906, DD01, NN-9423, LY-3437943, danuglipron, efpeglenatide, exenatide, noiiglutide, GMA105, HM-15136, noliglycopeptide (SHR-20004), carbetocin (LV-101), PYY-1562 (NNC0165-1562), PYY-1875 (NNC0165-1875), cotadutide/AM833 (NN9838, NNC-01740833), cotadutide (MEDI0382), LY3305677, pegapamodutide (LY2944876, OPK88003), NGM395, YH34160, CT-388, CT-868, SCO-267, SCO-792, diazoxide, tesofensine, namodenoson, ERX1000, AMG133, ASC41, Xlal, HDV Biotin, EMP16, metoprolol/tesofensine, RZL-012, CB4211, BI1356225, AMG171, NO-13065, bardoxolone methyl, HSG4112, YHC2129, YHC2134, KTX-0200, and S-723595.

### (Embodiment 3)

A pharmaceutical preparation characterized by combination of (A) and (B),
wherein (A) is a compound selected from the group consisting of compounds I-253, I-275, II-103, II-121, II-203, II-225, II-233, I'-6, I'-66, I'-124, I'-137, I'-142, I'-147, and I'-151, or a pharmaceutically acceptable salt thereof, and
(B) is at least one selected from orlistat, cetilistat, phentermine, mazindol, benzphetamine, amfepramone, methamphetamine, phentermine hydrochloride/topiramate, naltrexone hydrochloride/bupropion hydrochloride, liraglutide, semaglutide, setmelanotide (RM-493), metreleptin, topiramate, naltrexone, bupropion, tirzepatide, SCO-094, efinopegdutide (HM12525A), BI-456906, DD01, NN-9423, LY-3437943, danuglipron, PF07081532, LY-3502970, RGT-075, efpeglenatide, exenatide, noiiglutide, GMA105, HM-15136, noliglycopeptide (SHR-20004), carbetocin (LV-101), PYY-1562 (NNC0165-1562), PYY-1875 (NNC0165-1875), cotadutide/AM833 (NN9838, NNC-01740833), cotadutide (MEDI0382), LY3305677, pegapamodutide (LY2944876, OPK88003), NGM395, YH34160, CT-388, CT-868, SCO-267, SCO-792, diazoxide, tesofensine, namodenoson, ERX1000, AMG133, ASC41, Xlal, HDV Biotin, EMP16, metoprolol/tesofensine, RZL-012, CB4211, BI1356225, AMG171, NO-13065, bardoxolone methyl, HSG4112, YHC2129, YHC2134, KTX-0200, S-237648, and S-723595.

### (Embodiment 3')

A pharmaceutical preparation characterized by combination of (A) and (B),
wherein (A) is a compound selected from the group consisting of compounds I-253, I-275, II-103, II-121, II-203, II-225, II-233, I'-6, I'-66, I'-124, I'-137, I'-142, I'-147, and I'-151, or a pharmaceutically acceptable salt thereof, and
(B) is at least one selected from orlistat, cetilistat, phentermine, mazindol, benzphetamine, amfepramone, methamphetamine, phentermine hydrochloride/topiramate, naltrexone hydrochloride/bupropion hydrochloride, liraglutide, semaglutide, setmelanotide (RM-493), metreleptin, topiramate, naltrexone, bupropion, tirzepatide, SCO-094, efinopegdutide (HM12525A), BI-456906, DD01, NN-9423, LY-3437943, danuglipron, efpeglenatide, exenatide, noiiglutide, GMA105, HM-15136, noliglycopeptide (SHR-20004), carbetocin (LV-101), PYY-1562 (NNC0165-1562), PYY-1875 (NNC0165-1875), cotadutide/AM833 (NN9838, NNC-01740833), cotadutide (MEDI0382), LY3305677, pegapamodutide (LY2944876, OPK88003), NGM395, YH34160, CT-388, CT-868, SCO-267, SCO-792, diazoxide, tesofensine, namodenoson, ERX1000, AMG133, ASC41, Xlal, HDV Biotin, EMP16, metoprolol/tesofensine, RZL-012, CB4211, BI1356225, AMG171, NO-13065, bardoxolone methyl, HSG4112, YHC2129, YHC2134, KTX-0200, and S-723595.

### (Embodiment 4)

A pharmaceutical preparation characterized by combination of (A) and (B), wherein
(A) is a compound represented by formula: 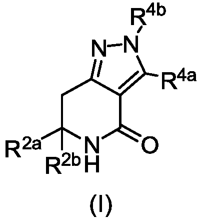 , or a pharmaceutically acceptable salt thereof,
   wherein
   R^{2a} and R^{2b} are taken together with an adjacent carbon atom to form a ring B, the ring B being represented by formula: 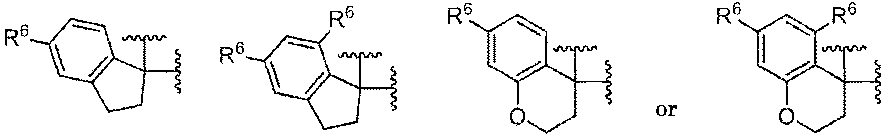 wherein R⁶s are each independently halogen, haloalkyl, or haloalkyloxy,
   R^{4a} is represented by formula: 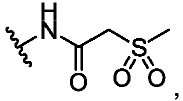 and
   R^{4b} is haloalkyloxyalkyl, a phenyl group optionally substituted with halogen, or 5- to 6-membered aromatic heterocyclyl optionally substituted with substituent group 6, wherein the substituent group β is halogen and alkyl, and
(B) is at least one selected from liraglutide, semaglutide, tirzepatide, BI-456906, danuglipron, PF07081532, LY-3502970, RGT-075, compounds represented by formulae: 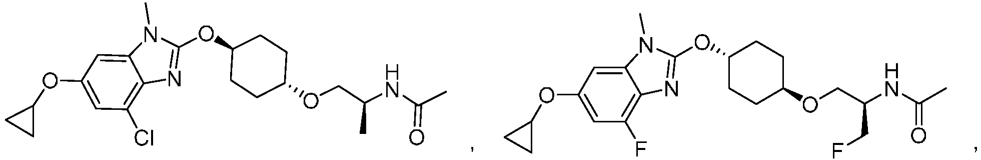 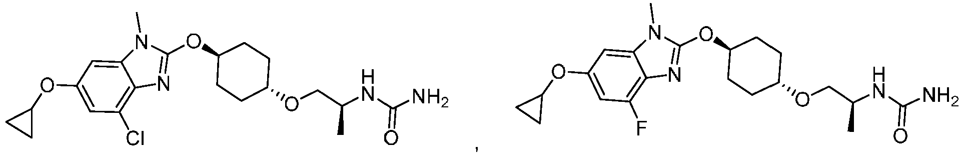 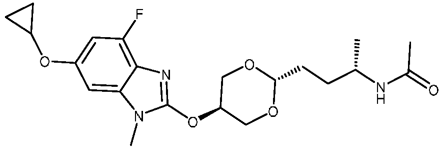 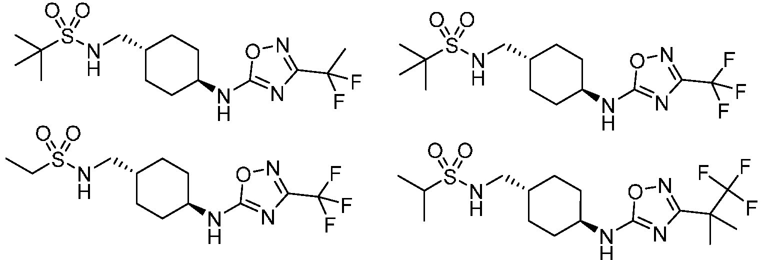 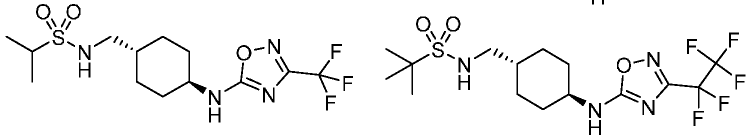 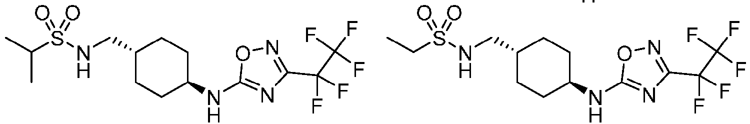 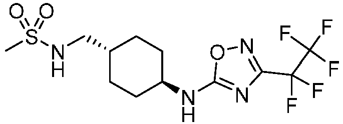 and pharmaceutically acceptable salts thereof.

### (Embodiment 4')

A pharmaceutical preparation characterized by combination of (A) and (B), wherein
(A) is a compound represented by formula: 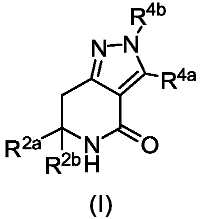 , or a pharmaceutically acceptable salt thereof,
   wherein
   R^{2a} and R^{2b} are taken together with an adjacent carbon atom to form a ring B, the ring B being represented by formula: 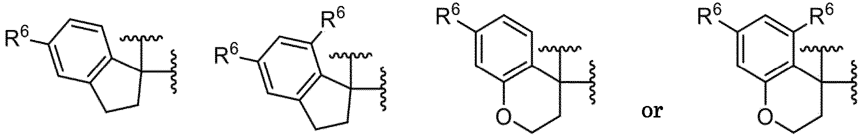 wherein R⁶s are each independently halogen, haloalkyl, or haloalkyloxy,
   R^{4a} is represented by formula: 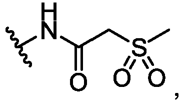 and
   R^{4b} is haloalkyloxyalkyl, a phenyl group optionally substituted with halogen, or 5- to 6-membered aromatic heterocyclyl optionally substituted with substituent group 6, wherein the substituent group B is halogen and alkyl, and
(B) is at least one selected from liraglutide, semaglutide, tirzepatide, BI-456906, danuglipron, a compound represented by formula: 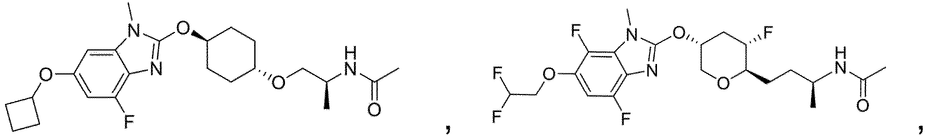 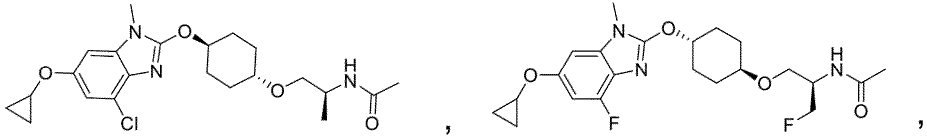 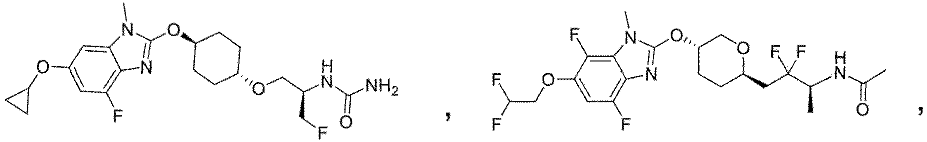 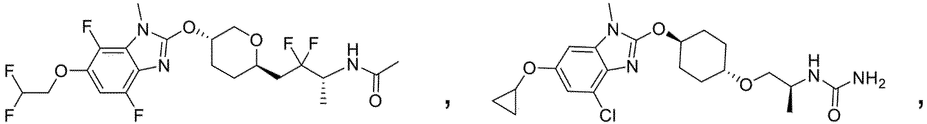 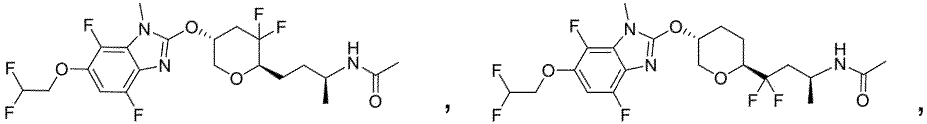 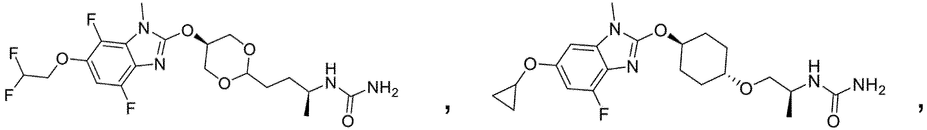 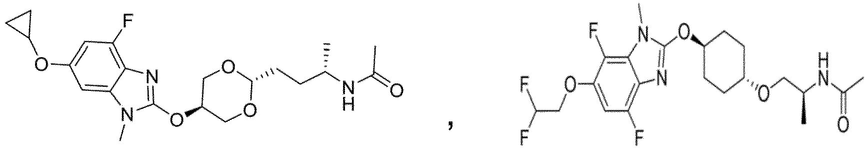 or 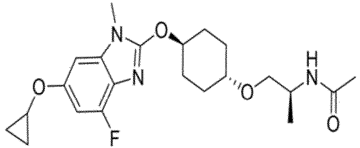 and pharmaceutically acceptable salts thereof.

### (Embodiment 5)

A pharmaceutical preparation characterized by combination of (A) and (B), wherein
(A) is a compound represented by formula: 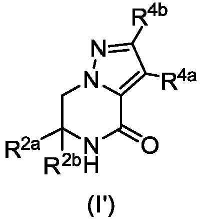 , or a pharmaceutically acceptable salt thereof,
   wherein
   R^{2a} and R^{2b} are taken together with an adjacent carbon atom to form a ring B, the ring B being represented by formula: 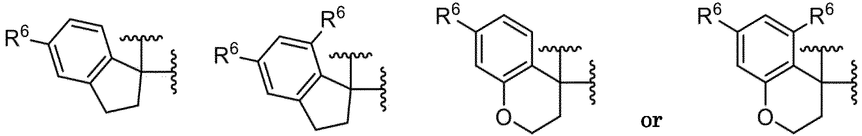 wherein R⁶s are each independently halogen, haloalkyl, or haloalkyloxy,
   R^{4a} is represented by formula: 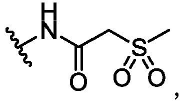 and
   R^{4b} is haloalkyloxyalkyl, a phenyl group optionally substituted with halogen, or 5- to 6-membered aromatic heterocyclyl optionally substituted with substituent group β, wherein the substituent group B is halogen and alkyl, and
(B) is at least one selected from liraglutide, semaglutide, tirzepatide, BI-456906, danuglipron, PF07081532, LY-3502970, RGT-075, compounds represented by formulae: 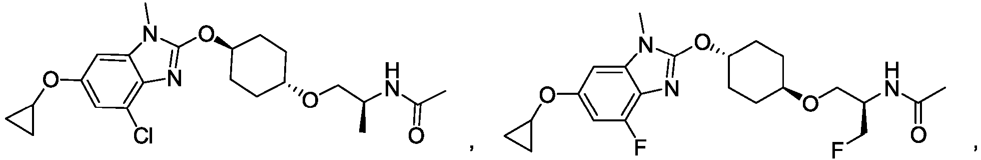 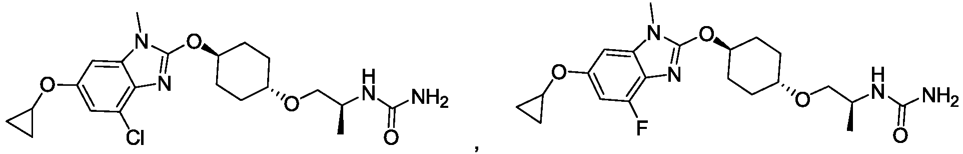 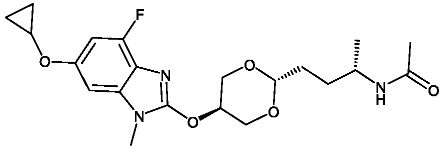 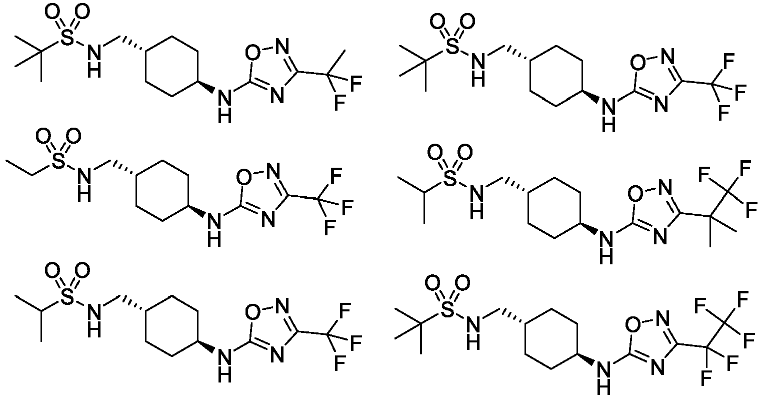 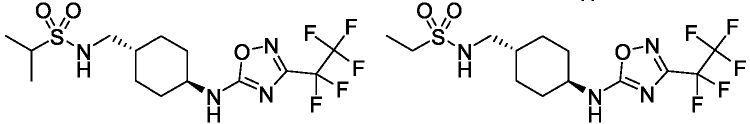 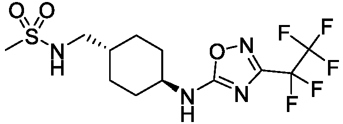 and pharmaceutically acceptable salts thereof.

### (Embodiment 5')

A pharmaceutical preparation characterized by combination of (A) and (B), wherein
(A) is a compound represented by formula: 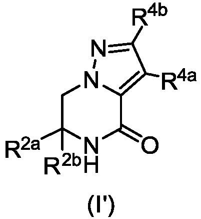 , or a pharmaceutically acceptable salt thereof,
   wherein
   R^{2a} and R^{2b} are taken together with an adjacent carbon atom to form a ring B, the ring B being represented by formula: 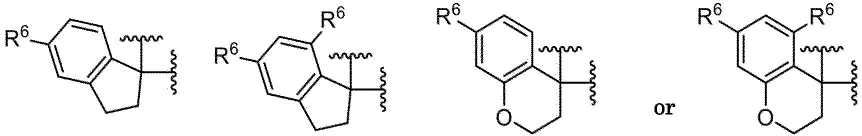 wherein R⁶s are each independently halogen, haloalkyl, or haloalkyloxy,
   R^{4a} is represented by formula: 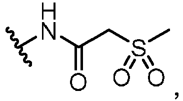 and
   R^{4b} is haloalkyloxyalkyl, a phenyl group optionally substituted with halogen, or 5- to 6-membered aromatic heterocyclyl optionally substituted with substituent group 6, wherein the substituent group B is halogen and alkyl, and
(B) is at least one selected from liraglutide, semaglutide, tirzepatide, BI-456906, danuglipron, a compound represented by formula: 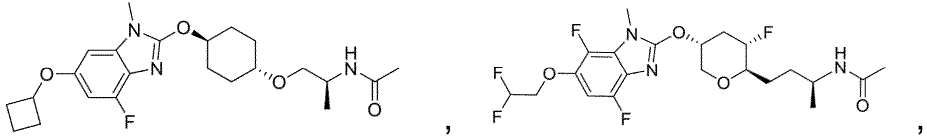 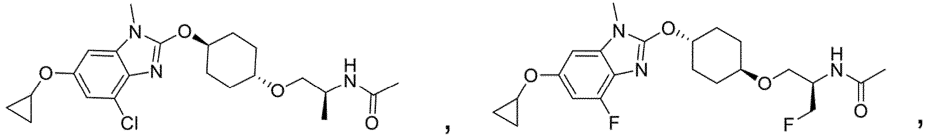 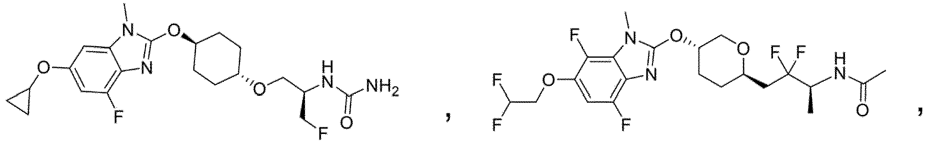 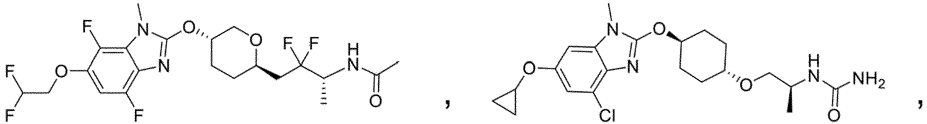 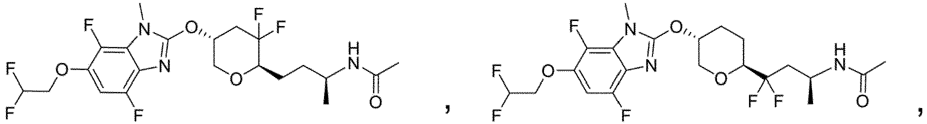 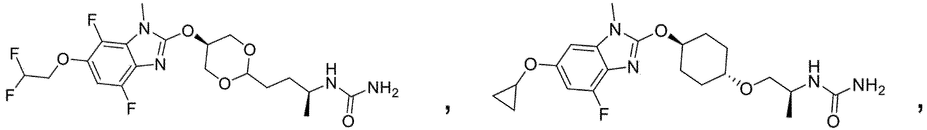 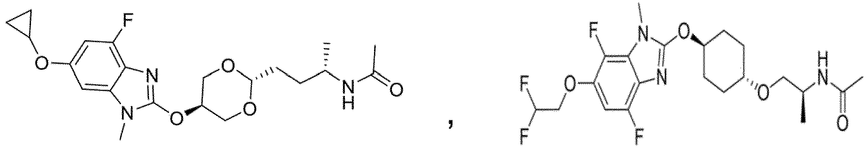 or 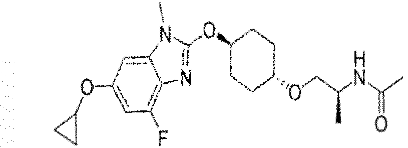 and pharmaceutically acceptable salts thereof.

### (Embodiment 6)

A pharmaceutical preparation characterized by combination of (A) and (B),
wherein (A) is a compound selected from the group consisting of compounds I-253, I-275, II-103, II-121, II-203, II-225, II-233, I'-6, I'-66, I'-124, I'-137, I'-142, I'-147, and I'-151, or a pharmaceutically acceptable salt thereof, and
(B) is at least one selected from liraglutide, semaglutide, tirzepatide, BI-456906, danuglipron, PF07081532, LY-3502970, RGT-075, compounds represented by formulae: 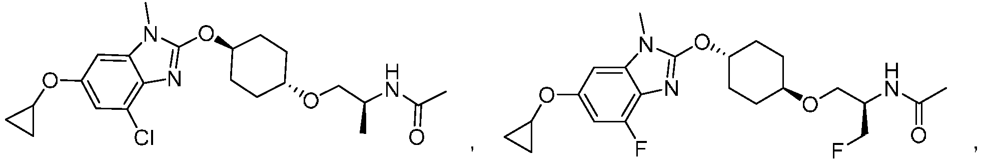 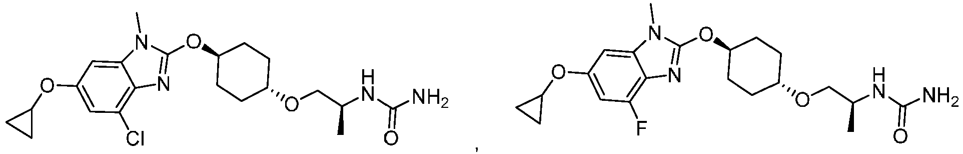 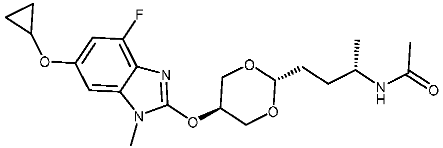 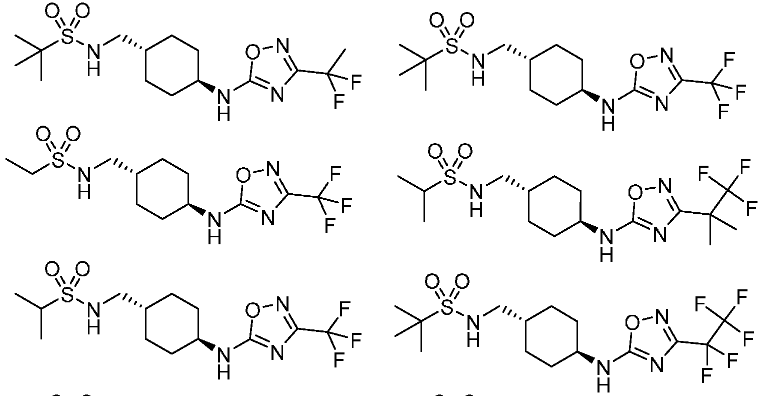 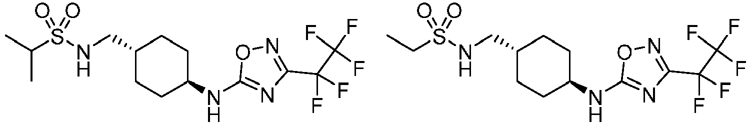 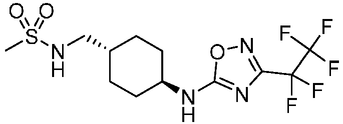 and pharmaceutically acceptable salts thereof.

### (Embodiment 6')

A pharmaceutical preparation characterized by combination of (A) and (B),
wherein (A) is a compound selected from the group consisting of compounds I-253, I-275, II-103, II-121, II-203, II-225, II-233, I'-6, I'-66, I'-124, I'-137, I'-142, I'-147, and I'-151, or a pharmaceutically acceptable salt thereof, and
(B) is at least one selected from liraglutide, semaglutide, tirzepatide, BI-456906, danuglipron, a compound represented by formula: 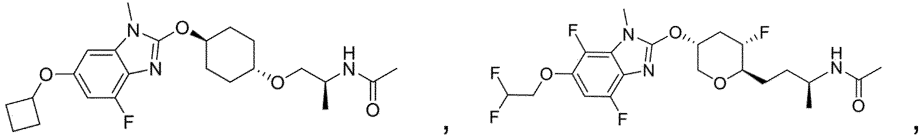 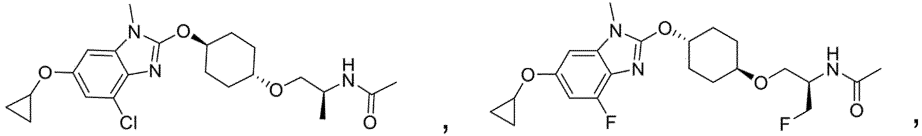 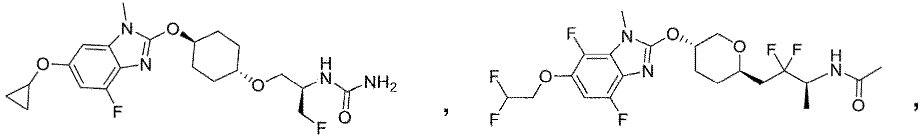 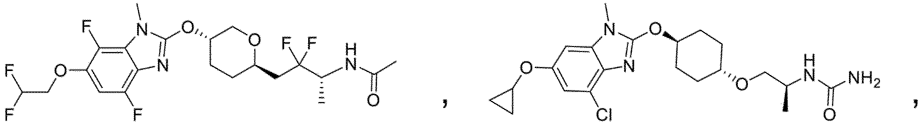 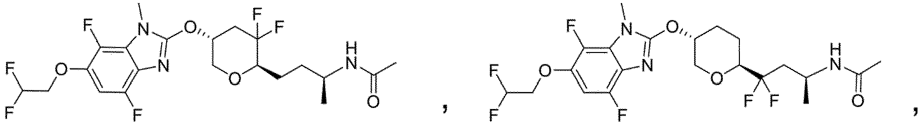 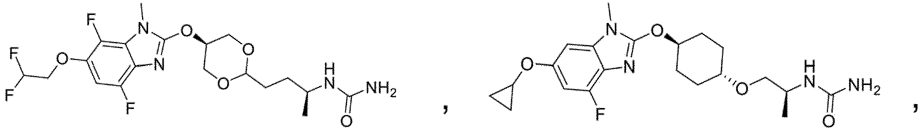 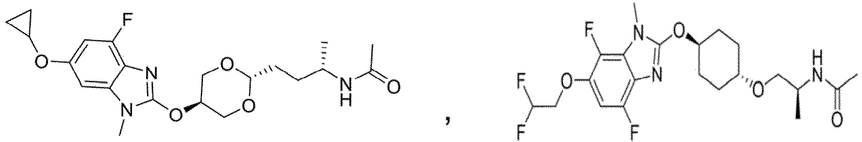 or 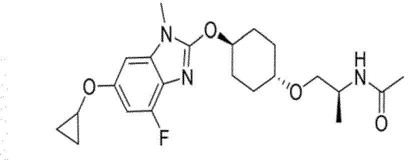 and pharmaceutically acceptable salts thereof.

The pharmaceutical preparation of the present invention can also be administered orally or parenterally. Examples of a parenteral administration method include percutaneous, subcutaneous, intravenous, intra-arterial, intramuscular, intraperitoneal, transmucosal, inhalation, transnasal, ocular instillation, ear instillation, and intravaginal administration.

In the case of oral administration, the pharmaceutical composition may be prepared into any dosage form that is commonly used, such as a solid preparation for internal use (for example, a tablet, a powder preparation, a granular preparation, a capsule, a pill, or a film preparation), or a liquid preparation for internal use (for example, a suspension, an emulsion, an elixir, a syrup, a limonade, a spirit preparation, an aromatic water preparation, an extraction, a decoction, or a tincture) and administered. The tablet may be a dragee, a film-coated tablet, an entericcoated tablet, a sustained release tablet, a troche, a sublingual tablet, a buccal tablet, a chewable tablet, or an orally disintegrating tablet; the powder preparation and granular preparation may be dry syrups; and the capsule may be a soft capsule, a microcapsule, or a sustained release capsule.

In the case of parenteral administration, the pharmaceutical composition can be suitably administered in any dosage form that is commonly used, such as an injectable preparation, an infusion, or a preparation for external use (for example, an eye drop, a nasal drop, an ear drop, an aerosol, an inhalant, a lotion, an impregnating agent, a liniment, a gargling agent, an enema, an ointment, a plaster, a jelly, a cream, a patch, a poultice, a powder preparation for external use, or a suppository). The injectable preparation may be an emulsion of O/W type, W/O type, O/W/O type, W/O/W type, or the like.

A pharmaceutical composition can be obtained by mixing an effective amount of the compound used in the pharmaceutical preparation of the present invention with various pharmaceutical additives appropriate for the dosage form, such as an excipient, a binder, a disintegrating agent, and a lubricating agent, as necessary. Furthermore, the pharmaceutical composition can be prepared into a pharmaceutical composition for use for a child, an elderly, a patient with a serious case, or a surgical operation, by appropriately changing the effective amount of the compound used in the pharmaceutical preparation of the present invention, the dosage form, and/or various pharmaceutical additives. For example, pediatric pharmaceutical compositions may be administered to patients who are neonates (younger than 4 weeks old after the birth), infants (4 weeks old to younger than 1 year old after the birth), children (1 year old or older and younger than 7 years old), infant children (7 years old or older and younger than 15 years old), or 15 to 18 years old. For example, the geriatric pharmaceutical compositions may be administered to patients who are 65 years old or older.

The dose of the pharmaceutical preparation of the present invention can be appropriately selected on the basis of the dose used on clinical. Furthermore, the mixing ratio of the compound represented by (A) and the concomitant medicament (B) can be appropriately selected in consideration of the subject of administration, administration route, target diseases, symptoms, combinations, and the like. For example, when the subject of administration is human, the concomitant medicament of (B) may be used in the range of 0.01 to 400 parts by weight with respect to 1 part by weight of the compound given as (A).

### [EXAMPLES]

Hereinafter, the present invention will be explained in more detail by way of Examples and Test Examples of the present invention, but the present invention is not limited by them. In addition, changes may be made without departing from the scope of the present invention. The compound names shown in the following Examples and Comparative Examples do not necessarily follow the IUPAC nomenclature.

NMR analyses achieved in Examples were performed by 300 MHz using DMSO-d₆ and CDCl₃.

### (Synthesis Example 1)

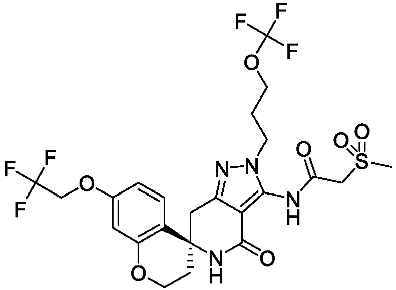

Compound II-121 was synthesized from a commercially available compound according to the method described in Patent Document 1.

¹H-NMR (CDCl₃) δ: 2.09-2.18 (m, 2H), 2.29-2.36 (m, 2H), 3.03 (d, J = 16.3 Hz, 1H), 3.21 (s, 3H), 3.31 (d, J = 16.3 Hz, 1H), 3.99-4.35 (m, 10H), 5.92 (s, 1H), 6.40 (d, J = 2.4 Hz, 1H), 6.60 (dd, J = 8.8, 2.4 Hz, 1H), 7.41 (d, J = 8.8 Hz, 1H), 9.56 (s, 1H).

The following compounds were synthesized from commercially available compounds according to the methods described in Patent Documents 1 to 4.

**[Table 1]**

| No. | Structure | No. | Structure |
|---|---|---|---|
| I-008 | 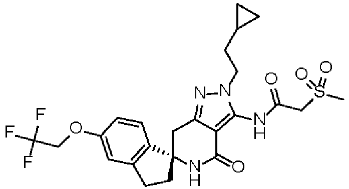 | I-253 | 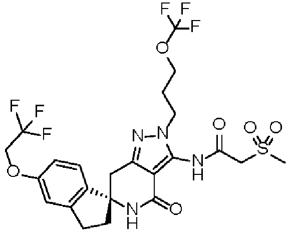 |
| I-023 | 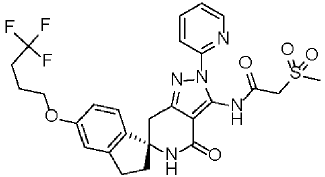 | I-275 | 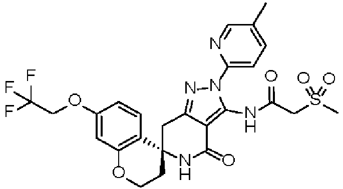 |
| I-034 | 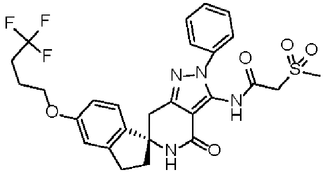 | I-276 | 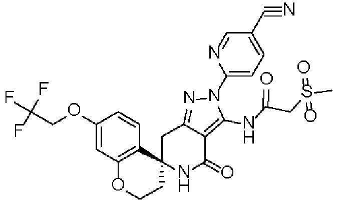 |
| I-190 | 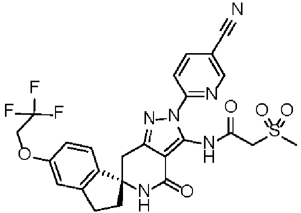 | II-093 | 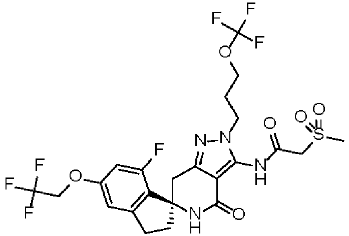 |
| I-212 | 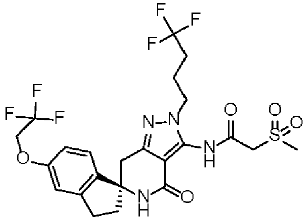 | II-103 | 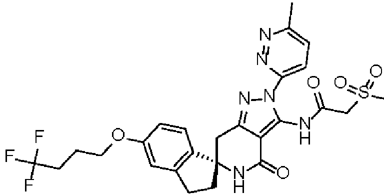 |
| I-236 | 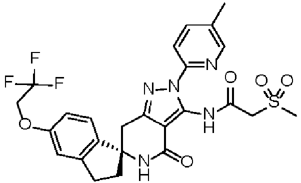 | II-151 | 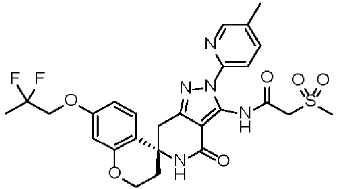 |

**[Table 2]**

| No. | Structure | No. | Structure |
|---|---|---|---|
| II-168 | 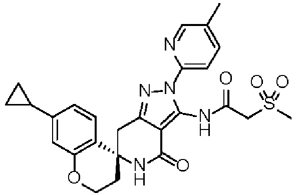 | I'-006 | 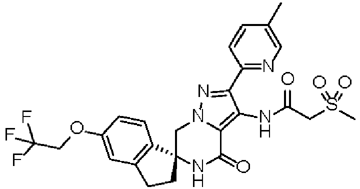 |
| II-174 | 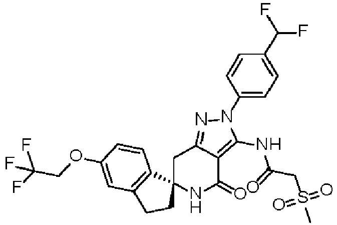 | I'-020 | 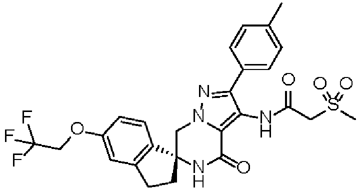 |
| II-203 | 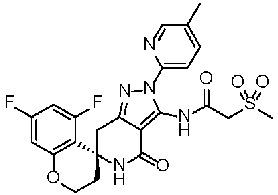 | I'-033 | 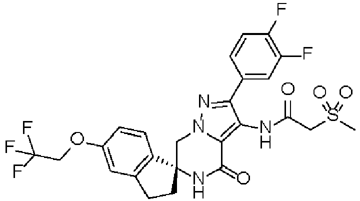 |
| II-225 | 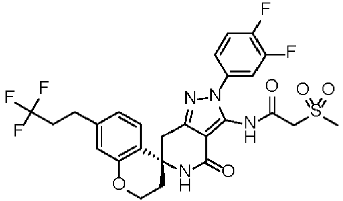 | I'-046 | 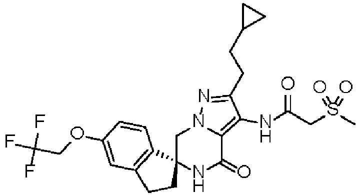 |
| II-233 | 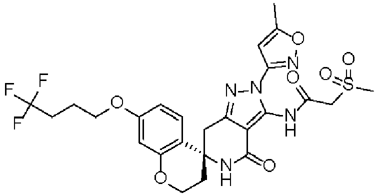 | I'-058 | 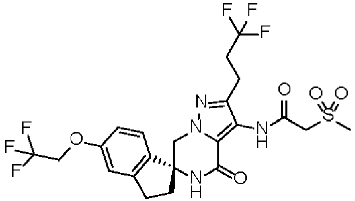 |
| II-295 | 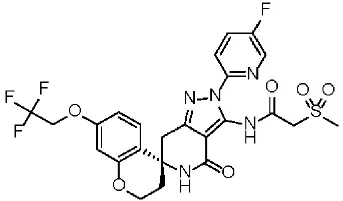 | I'-066 | 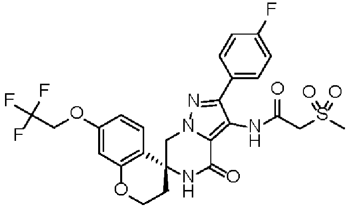 |

**[Table 3]**

| No. | Structure | No. | Structure |
|---|---|---|---|
| I'-079 | 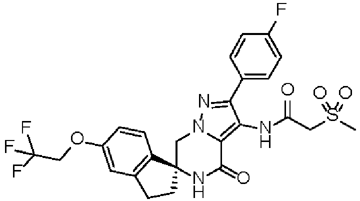 | I'-136 | 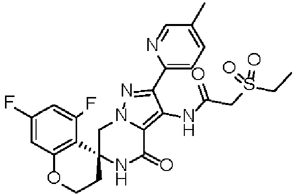 |
| I'-123 | 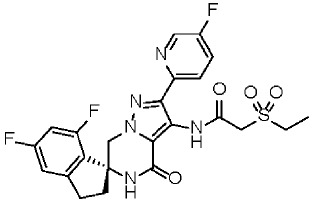 | I'-137 | 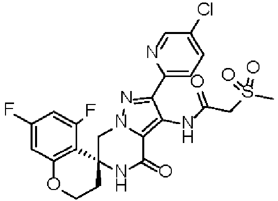 |
| I'-124 | 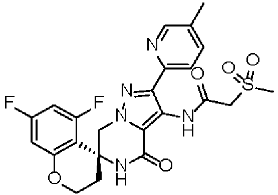 | I'-139 | 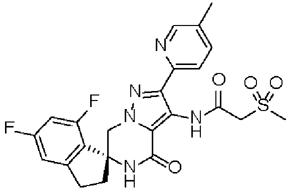 |
| I'-126 | 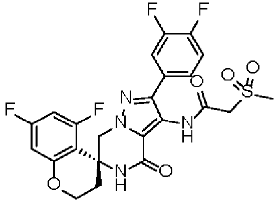 | I'-141 | 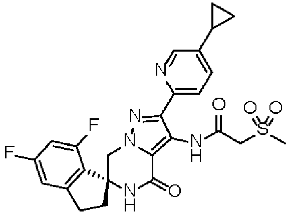 |
| I'-130 | 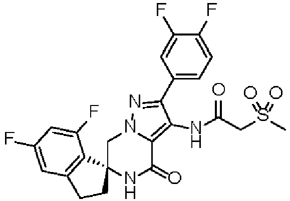 | I'-142 | 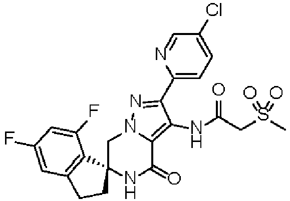 |
| I'-133 | 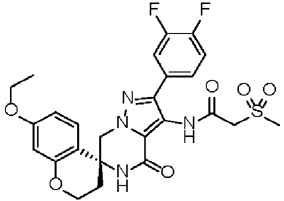 | I'-143 | 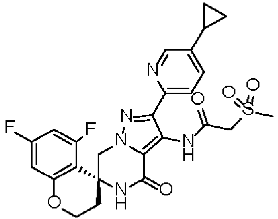 |

**[Table 4]**

| No. | Structure | No. | Structure |
|---|---|---|---|
| I'-145 | 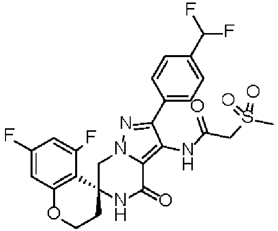 | I'-151 | 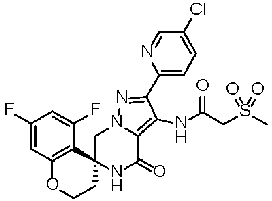 |
| I'-147 | 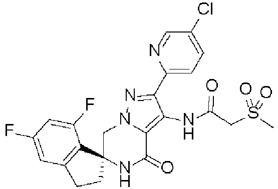 | I'-153 | 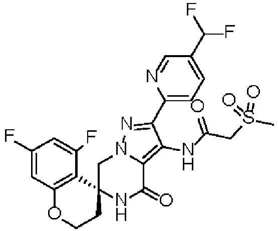 |

Physical data on each compound were shown below.
In the table, "MS" represents the mass (M + H) in LC/MS. [Measurement conditions A]
Column: ACQUITY UPLC (registered trademark) BEH C18 (1.7 µm i.d.2.1 x 50 mm) (Waters)
Flow rate: 0.8 mL/min
UV detection wavelength: 254 nm
Mobile phase: [A] was 0.1% formic acid-containing aqueous solution, and [B] was 0.1% formic acid-containing acetonitrile solution.
Linear gradient of 5% to 100% solvent [B] was performed in 3.5 minutes, and then 100% solvent [B] was kept for 0.5 minutes.

### [Measurement conditions B]

Column: Shim-pack XR-ODS (2.2 µm, i.d.50 x 3.0 mm) (Shimadzu)
Flow rate: 1.6 mL/min
UV detection wavelength: 254 nm
Mobile phase: [A] was 0.1% formic acid-containing aqueous solution, and [B] was 0.1% formic acid-containing acetonitrile solution.
Gradient: linear gradient of 10% to 100% solvent [B] was performed in 3 minutes, and 100% solvent [B] was kept for 0.5 minutes.

### [Measurement conditions C]

Column: ACQUITY UPLC (registered trademark) BEH C18 (1.7 µm i.d.2.1 x 50 mm) (Waters)
Flow rate: 0.55 mL/min
UV detection wavelength: 254 nm
Mobile phase: [A] was 0.1% formic acid-containing aqueous solution, and [B] was 0.1% formic acid-containing acetonitrile solution.
Gradient: linear gradient of 5% to 100% solvent [B] for 3 minutes was performed, and then 100% solvent [B] was maintained for 0.5 minute.

**[Table 5]**

| No. | MS | No. | MS | No. | MS | No. | MS | No. | MS | No. | MS | No. | MS |
|---|---|---|---|---|---|---|---|---|---|---|---|---|---|
| I-008 | 541 | I-253 | 599 | II-151 | 576 | II-295 | 584 | I'-066 | 583 | I'-133 | 547 | I'-143 | 544 |
| I-023 | 578 | I-275 | 580 | II-168 | 522 | I'-006 | 564 | I'-079 | 567 | I'-136 | 532 | I'-145 | 553 |
| I-034 | 577 | I-276 | 599 | II-174 | 599 | I'-020 | 563 | I'-123 | 520 | I'-137 | 538 | I'-147 | 522 |
| I-190 | 575 | II-093 | 617 | II-203 | 518 | I'-033 | 585 | I'-124 | 518 | I'-139 | 502 | I'-151 | 538 |
| I-212 | 583 | II-103 | 593 | II-225 | 599 | I'-046 | 541 | I'-126 | 539 | I'-141 | 528 | I'-153 | 554 |
| I-236 | 564 | II-121 | 615 | II-233 | 598 | I'-058 | 569 | I'-130 | 523 | I'-142 | 522 | | |

NMR analysis of each Example was performed by 300 MHz using DMSO-d₆ or CDCl₃.

**[Table 6]**

| No. | NMR |
|---|---|
| I-008 | 1H-NMR (CDCl3) δ: 0.00-0.02 (m, 2H), 0.39-0.44 (m, 2H), 0.61-0.68 (m, 1H), 1.76 (q, J = 7.2 Hz, 2H), 2.10-2.17 (m, 1H), 2.38-2.44 (m, 1H), 2.86-3.07 (m, 5H), 3.24 (s, 3H), 4.09 (t, J = 7.3 Hz, 2H), 4.21 (d, J = 14.3 Hz, 1H), 4.28-4.34 (m, 3H), 5.97 (s, 1H), 6.78-6.83 (m, 2H), 7.19 (d, J = 8.3 Hz, 1H), 9.60 (s, 1H). |
| I-023 | 1H-NMR (CDCl3) δ: 2.02-2.09 (m, 2H), 2.21-2.47 (m, 4H), 2.88-3.01 (m, 2H), 3.05 (d, J = 15.6 Hz, 1H), 3.18 (d, J = 15.8 Hz, 1H), 3.24 (s, 3H), 4.01 (t, J = 5.9 Hz, 2H), 4.17 (s, 2H), 5.63 (s, 1H), 6.78-6.80 (m, 2H), 7.27-7.30 (m, 2H), 7.87-7.95 (dd, J = 13.1, 8.5 Hz, 2H), 8.48 (br-d, J = 5.0 Hz, 1H), 11.15 (s, 1H). |
| I-034 | 1H-NMR (CDCl3) δ: 1.95-2.06 (2H, m), 2.19-2.32 (3H, m), 2.44-2.54 (1H, m), 2.90 (3H, s), 2.91-3.04 (2H, m), 3.09 (1H, d, J = 15.8 Hz), 3.18 (1H, d, J = 15.8 Hz), 3.88-3.97 (2H, m), 4.02 (1H, d, J = 14.3 Hz), 4.22 (1H, d, J = 14.3 Hz), 6.12 (1H, s), 6.66-6.72 (1H, m), 6.76-6.79 (1H, m), 7.18 (1H, d, J = 8.3 Hz), 7.35-7.52 (5H, m), 9.66 (1H, s). |
| I-190 | 1H-NMR (CDCl3) δ: 2.22-2.30 (m, 1H), 2.41-2.47 (dd, J = 12.3, 8.3 Hz, 1H), 2.90-3.21 (m, 7H), 4.18 (s, 2H), 4.35 (q, J = 8.1 Hz, 2H), 5.69 (s, 1H), 6.83-6.87 (m, 2H), 7.31 (d, J = 8.3 Hz, 1H), 8.07-8.14 (m, 2H), 8.78 (m, 1H), 10.73 (s, 1H). |
| I-275 | 1H-NMR (CDCI3) δ: 2.19-2.31(m, 2H), 2.40(s, 3H), 3.11(d, J=16.3 Hz, 1H), 3.22(s, 3H), 3.41 (d, J=16.3 Hz, 1H), 4.14(s, 2H), 4.23-4.25(m, 2H), 4.33(q, J=8.0 Hz, 2H), 5.59(s, 1H), 6.44(d, J=2.6 Hz, 1H), 6.62(dd, J=8.8, 2.6 Hz, 1H), 7.49(d, J=8.8 Hz, 1H), 7.69-7.72(m, 1H), 7.81(d, J=8.3 Hz, 1H), 8.30(br-s, 1H), 11.19(s, 1H). |
| II-168 | 1H-NMR (CDC)3) δ: 0.68-0.72(m, 2H), 0.95-1.00(m, 2H), 1.81-1.88(m, 1H), 2.19-2.28(m, 2H), 2.40(s, 3H), 3.11(d, J=16.3 Hz, 1H), 3.23(s, 3H), 3.42(d, J=16.3 Hz, 1H), 4.14(s, 2H), 4.20-4.22(m, 2H), 5.61(s, 1H), 6.57(d, J=1.8 Hz, 1H), 6.73(dd, J=8.2, 1.8 Hz, 1H), 7.42(d, J=8.2 Hz, 1H), 7.70 (dd, J=8.4, 1.8 Hz, 1H), 7.81(d, J=8.4 Hz, 1H), 8.30(s, 1H), 11.17(s, 1H). |
| II-203 | 1H-NMR (CDCl3) δ: 2.19-2.26(m, 1H), 2.32-2.40(m, 4H), 2.99(d, J=16.2 Hz, 1H), 3.22(s, 3H), 3.81(d, J=16.2 Hz, 1H), 4.15-4.20(m, 4H), 5.77(s, 1H), 6.47-6.52(m, 2H), 7.69-7.71(m, 1H), 7.81(d, J=8.3 Hz, 1H), 8.30(br-s, 1H). |
| I'-006 | 1H-NMR (CDCl3) δ: 2.26-2.34 (m, 1H), 2.38 (s, 3H), 2.53-2.59 (m, 1H), 2.94-3.09 (m, 2H), 3.23 (s, 3H), 4.12 (s, 2H), 4.32-4.40 (m, 4H), 6.05 (s, 1H), 6.85-6.89 (m, 2H), 7.26-7.29 (m, 1H), 7.57 (br-d, 1H, J = 8.2 Hz), 7.84 (d, 1H, J = 8.0 Hz), 8.47 (br-s, 1H), 10.15 (s, 1H). |
| I'-046 | 1H-NMR (CDCl3) δ: 0.03-0.06 (m, 2H), 0.39-0.43 (m, 2H), 0.69-0.76 (m, 1H), 1.52-1.57 (m, 2H), 2.16-2.24 (m, 1H), 2.50-2.55 (m, 1H), 2.79-2.82 (m, 2H), 2.91-3.07 (m, 2H), 3.22 (s, 3H), 4.12-4.22 (m, 4H), 4.35 (q, 2H, J = 8.0 Hz), 6.25 (s, 1H), 6.84-6.87 (m, 2H), 7.17 (d, 1H, J = 8.4 Hz), 8.72 (s, 1H). |
| I'-126 | 1H-NMR (DMSO-D6) δ: 2.01-2.07 (m, 1H), 2.18-2.23 (m, 1H), 3.14 (s, 3H), 4.19-4.38 (m, 4H), 4.72 (dd, 2H, J = 22.1, 13.6 Hz), 6.71 (d, 1H, J = 10.0 Hz), 6.87-6.93 (m, 1H), 7.44 (dd, 1H, J = 19.1, 8.7 Hz), 7.63-7.66 (m, 1H), 7.73-7.78 (m, 1H), 8.96 (s, 1H), 10.14 (s, 1H). |
| I'-133 | 1H-NMR(CDCl3) δ: 1.40 (t, 3H, J = 7.0 Hz), 2.21-2.30 (m, 2H), 3.10 (s, 3H), 3.99 (q, 2H, J = 7.0 Hz), 4.09 (d, 1H, J = 14.1 Hz), 4.20-4.29 (m, 3H), 4.50 (dd, 2H, J = 22.5, 13.6 Hz), 6.41 (d, 1H, J = 2.4 Hz), 6.44 (s, 1H), 6.57 (dd, 1H, J = 8.7, 2.3 Hz), 7.19 (q, 1H, J = 8.9 Hz), 7.29 (d, 1H, J = 8.8 Hz), 7.47 (d, 1H, J = 8.5 Hz), 7.56 (t, 1H, J = 9.4 Hz), 8.99 (s, 1H). |
| I'-137 | 1H-NMR (DMSO-D6) δ: 2.04-2.09 (m, 1H), 2.20-2.25 (d, 1H, J = 18.4 Hz), 3.19 (s, 3H), 4.21-4.39 (m, 4H), 4.74 (dd, 2H, J = 17.6, 13.8 Hz), 6.72 (d, 1H, J = 9.9 Hz), 6.88-6.93 (m, 1H), 7.85 (d, 1H, J = 8.5 Hz), 7.96 (dd, 1H, J = 8.6, 2.2 Hz), 8.60 (d, 1H, J = 2.0 Hz), 8.98 (s, 1H), 10.09 (s, 1H). |
| I'-141 | 1H-NMR (CDC)3) δ: 0.78 (d, 2H, J = 4.8 Hz), 1.08 (d, 2H, J = 7.4 Hz), 1.94 (t, 1H, J = 4.5 Hz), 2.37-2.44 (m, 1H), 2.54-2.61 (m, 1H), 2.97-3.04 (m, 1H), 3.10-3.17 (m, 1H), 3.23 (s, 3H), 4.12 (s, 2H), 4.34 (d, 1H, J = 12.8 Hz), 4.76 (d, 1H, J = 12.8 Hz), 6.13 (s, 1H), 6.75 (t, 1H, J = 9.0 Hz), 6.86 (d, 1H, J = 7.3 Hz), 7.38 (d, 1H, J = 7.5 Hz), 7.83 (d, 1H, J = 8.2 Hz), 8.44 (s, 1H). |
| I'-142 | 1H-NMR (CDCl3) δ: 2.37-2.44 (m, 1H), 2.54-2.61 (m, 1H), 2.97-3.05 (m, 1H), 3.12-3.18 (m, 1H), 3.22 (s, 3H), 4.12 (s, 2H), 4.35 (d, 1H, J = 12.9 Hz), 4.77 (d, 1H, J = 12.8 Hz), 6.17 (s, 1H), 6.75 (t, 1H, J = 9.0 Hz), 6.86 (d, 1H, J = 7.5 Hz), 7.75 (d, 1H, J = 8.4 Hz), 7.91 (d, 1H, J = 8.4 Hz), 8.60 (s, 1H), 9.77 (s, 1H). |
| I'-147 | 1H-NMR (CDCl3) δ: 2.37-2.44 (m, 1H), 2.54-2.60 (m, 1H), 2.97-3.05 (m, 1H), 3.11-3.18 (m, 1H), 3.22 (s, 3H), 4.12 (s, 2H), 4.35 (d, 1H, J = 12.9 Hz), 4.77 (d, 1H, J = 12.9 Hz), 6.17 (s, 1H), 6.75 (t, 1H, J = 9.5 Hz), 6.86 (d, 1H, J = 7.3 Hz), 7.74 (dd, 1H, J = 8.5, 2.5 Hz), 7.91 (d, 1H, J = 8.5 Hz), 8.60 (d, 1H, J = 2.0 Hz), 9.77 (s, 1H). |
| I'-151 | 1H-NMR (CDCl3) δ: 2.28-2.38 (m, 2H), 3.20 (s, 3H), 4.11 (s, 2H), 4.23 (t, 2H, J = 5.2 Hz), 4.39 (d, 1H, J = 13.3 Hz), 4.97 (d, 1H, J = 13.2 Hz), 6.17 (s, 1H), 6.52-6.57 (m, 2H), 7.75 (dd, 1H, J = 8.5, 2.3 Hz), 7.92 (d, 1H, J = 8.4 Hz), 8.60 (d, 1H, J = 1.9 Hz). |

In addition, as the "at least one agent selected from the group consisting of agents having anti-obesity effect, agents for controlling blood glucose level, agents for controlling cholesterol and/or triglyceride in blood, and agents for controlling blood pressure" of (B), a commercially available agent or an agent synthesized by the method described in Patent Documents was used.

### Test Example 1: Measurement of Human MGAT2 Inhibitory Activity

Solutions of the compounds of the present invention in DMSO were each aliquoted into 0.2-pL portions in a 384-well polystyrene microplate produced by Corning Incorporated, and 5 µL of an enzyme solution prepared with an assay buffer (100 mmol/L phosphate buffer (pH 7.4) containing 2 mmol/L DTT) and 5 µL of a substrate solution (100 mmol/L phosphate buffer (pH 7.4), 30 µmol/L 2-Oleoylglycerol, 10 µmol/L Oleoyl-CoA) were added thereto, and the resultant was stirred and centrifuged, and incubated in a moist chamber at room temperature for 1 hour. After enzymatic reaction, 50 µL of a quenching solution (containing 0.2 µmol/L Diolein-d5, 0.4% formic acid, and 50% isopropanol) containing Internal Standard (IS) was added to terminate the reaction, and the resultant was sealed in a plate produced by Shimadzu GLC Ltd., and then stirred and centrifuged, and measurement was performed by using an electrospray ionization method with a RapidFire360 and Agilent 6550 Q-TOF mass spectrometer. Diolein as a reaction product (P) of 2-Oleoylglycerol as the substrate and an ammonium adduct ion of the IS were detected, and the peak intensity ratio, P/IS, was calculated from the peak heights to evaluate the inhibitory activity. Inhibitory activities with/without addition of enzyme were defined as Control (+)/Control (-), respectively, and the respective % inhibitions were defined as 0% inhibition and 100% inhibition. The inhibitory activity was calculated from the formula below with TIBCO Spotfire (produced by TIBCO Software Inc.) with the peak intensity ratio, P/IS, at the time of addition of the compound of the present invention being as Sample: Inhibitory activity(%)=[1-{Sample - Control (-)}/{Control(+)-Control (-)}]*100

The inhibitory activity results of the compounds of the present invention are shown in the following table. IC₅₀ (nM) in the tables indicates a concentration exhibiting 50% enzyme inhibition.

**[Table 7]**

| No. | IC50 (nM) | No. | IC50 (nM) | No. | IC50 (nM) | No. | IC50 (nM) | No. | IC50 (nM) | No. | IC50 (nM) | No. | IC50 (nM) |
|---|---|---|---|---|---|---|---|---|---|---|---|---|---|
| I-008 | 2.78 | I-253 | 1.40 | II-151 | 2.05 | II-295 | <1 | I'-066 | 0.85 | I'-133 | 1.39 | I'-143 | 3.43 |
| I-023 | 6.50 | I-275 | 2.00 | II-168 | 1.88 | I'-006 | 2.25 | I'-079 | 1.13 | I'-136 | 4.97 | I'-145 | 2.90 |
| I-034 | 18.00 | I-276 | 4.40 | II-174 | 2.16 | I'-020 | 1.97 | I'-123 | 8.48 | I'-137 | 1.53 | I'-147 | 3.50 |
| I-190 | 2.90 | II-093 | 1.21 | II-203 | 2.98 | I'-033 | 0.73 | I'-124 | 6.50 | I'-139 | 15.80 | I'-151 | 5.33 |
| I-212 | 6.90 | II-103 | 1.61 | II-225 | 1.14 | I'-046 | 1.17 | I'-126 | 1.59 | I'-141 | 6.42 | I'-153 | 2.23 |
| I-236 | 2.80 | II-121 | 2.91 | II-233 | 0.22 | I'-058 | 9.78 | I'-130 | 4.51 | I'-142 | 2.88 | | |

### Test Example 2: Anti-obesity Effect Test

The anti-obesity effect of the compound and/or agent according to the present invention was examined by using C57BL/6j mice (DIO mice) provided with a high-fat diet (TestDiet; 58Y1).

Five-week-old male C57BL/6j mice (CLEA Japan, Inc.) were purchased, and grown with feeding of a high-fat diet under 12-hour light-dark cycles for 4 weeks to produce DIO mice. A medium (0.5% HPMC and physiological saline) was administered once a day from 3 weeks before administration of the compound. Randomization was performed for grouping (n = 9 to 10) on the basis of body weight and change in food consumption during the period of administration for conditioning. From Day 0 to Day 35, gavage of vehicle (0.5% HPMC and physiological saline) (hereinafter, Control group), gavage of Compound (A) according to the present invention (hereinafter, (A)-administration group), subcutaneous administration of GLP-1 formulation (liraglutide or semaglutide) as Compound (B) according to the present invention (hereinafter, (B)-administration group), or both (hereinafter, the combined administration group) were performed once a day. The GLP-1 formulation was formulated in an increasing dose, and liraglutide and semaglutide were administered at 4 to 20 pg/kg and 3 to 15 µg/kg, respectively, once a day. During the administration period, daily body weight measurement and food consumption measurement once a week were performed.

### (Result)

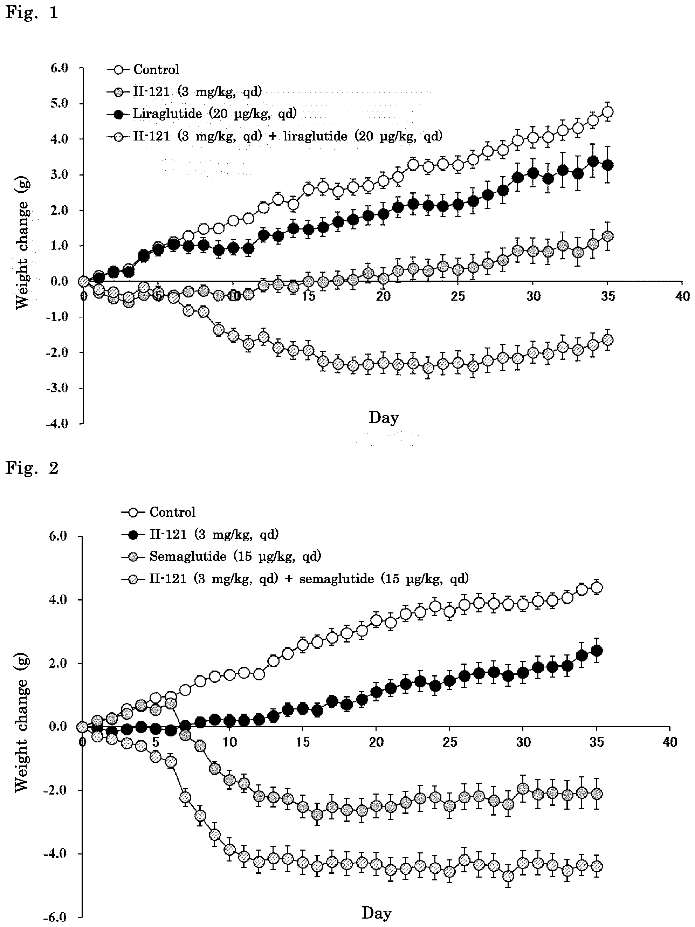

The daily weight changes are shown in Figs. 1 and 2. The rates of decrease in body weight relative to the control group at the time of final administration were as follows.

**[Table 8]**

| Administration group | Rate of decrease in body weight relative to control group | Administration group | Rate of decrease in body weight relative to control group |
|---|---|---|---|
| (A) 11-121 | 10.0% | (A) II-121 | 5.5% |
| (B) Liraglutide | 4.2% | (B) Semaglutide | 18.0% |
| II-121 + liraglutide | 18.1% | II-121 + semaglutide | 24.8% |

Changes in food intake are shown below.

**[Table 9]**

| Administration group | Rate of decrease in food consumption relative to control group | | | | |
|---|---|---|---|---|---|
| | 1st week of administration | 2nd week of administration | 3rd week of administration | 4th week of administration | 5th week of administration |
| (A) 11-121 | 18.9% | 12.7% | 9.9% | 8.5% | 7.5% |
| (B) Liraglutide | 4.6% | 9.4% | 3.1% | 2.4% | 1.5% |
| II-121 + liraglutide | 23.0% | 30.8% | 21.7% | 13.5% | 9.6% |

**[Table 10]**

| Administration group | Rate of decrease in food consumption relative to control group | | | | |
|---|---|---|---|---|---|
| | let week of administration | 2nd week of administration | 3rd week of administration | 4th week of administration | 5th week of administration |
| (A) II-121 | 15.7% | 10.5% | 7.3% | 3.9% | 1.6% |
| (B) Semaglutide | 8.5% | 33.1% | 18.2% | 10.9% | 6.0% |
| II-121 + semaglutide | 30.8% | 42.7% | 27.3% | 20.7% | 15.8% |

From the above results, in the combined administration group, a synergistic body weight gain suppressing effect and a food intake suppressing effect were observed as compared with the group in which only one of the compounds was administered ((A)-administration group or (B)-administration group).

From this, it was found that when (A) the MGAT2 inhibitor which is the compound according to the present invention is combined with (B) the GLP-1 formulation, an excellent synergistic weight gain suppressing effect and an excellent food intake suppressing effect are exhibited.

From these test results, the pharmaceutical preparation of the present invention can be a pharmaceutical preparation useful for the prevention and/or treatment of obesity or obesity-related diseases.

### Formulation Example

The following formulation examples are merely examples and not intended to limit the scope of the invention.

The compound used in the present invention can be administered as a pharmaceutical composition by any conventional route, in particular enterally, for example, orally, for example, in the form of tablets or capsules, or parenterally, for example, in the form of injectable solutions or suspensions, topically, for example, in the form of lotions, gels, ointments or creams, or in a nasal or suppository form. Pharmaceutical compositions comprising a compound used in the present invention in free form or in a pharmaceutically acceptable salt form in association with at least one pharmaceutically acceptable carrier or diluent can be manufactured in a conventional manner by mixing, granulating or coating methods. For example, the oral composition can be a tablet, a granular preparation, or a capsule, each containing an excipient, a disintegrating agent, a binder, a lubricating agent, and the like, as well as an active ingredient and the like. Furthermore, the composition for injection can be prepared as a solution or a suspension, may be sterilized, and may contain a preservative, a stabilizer, a buffering agent, and the like.

### [INDUSTRIAL APPLICABILITY]

The pharmaceutical preparation of the present invention can be a pharmaceutical preparation useful for the prevention and/or treatment of obesity or obesity-related diseases.

## Claims

1. A pharmaceutical preparation **characterized by** combination of (A) and (B), wherein
(A) is a compound represented by formula: 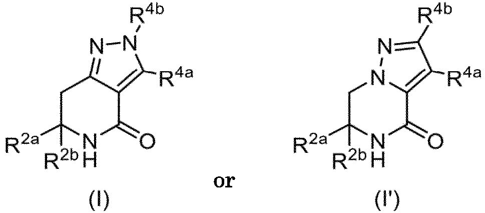 , or a pharmaceutically acceptable salt thereof,
wherein
R^{2a} and R^{2b} are taken together with an adjacent carbon atom to form a ring B, the ring B being represented by formula: 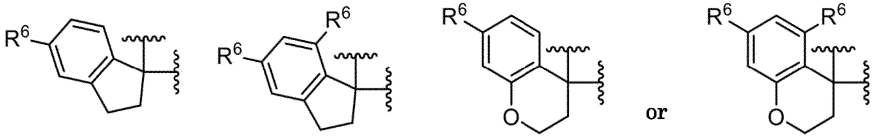 wherein R⁶s are each independently halogen, haloalkyl, alkyloxy, haloalkyloxy, or cyclopropanyl,
R^{4a} is represented by formula: 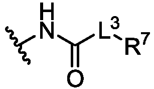
wherein
L³ is alkylene, and
R⁷ is alkylsulfamoyl, and
R^{4b} is alkyl optionally substituted with substituent group α, a phenyl group optionally substituted with substituent group 6, or 5- to 6-membered aromatic heterocyclyl optionally substituted with substituent group β,
wherein
the substituent group α is halogen, haloalkyloxy, and cyclopropanyl, and
the substituent group β is halogen, cyano, alkyl, haloalkyl, and cyclopropanyl, and
(B) is at least one drug selected from the group consisting of a drug having anti-obesity effects, a drug for controlling blood glucose level, a drug for controlling cholesterol and/or triglyceride in blood, and a drug for controlling blood pressure.

2. The pharmaceutical preparation according to claim 1,
wherein (A) is a compound selected from the group consisting of compounds I-8, I-23, I-34, I-190, I-212, I-236, I-253, I-275, I-276, II-93, II-103, II-121, II-151, II-168, II-174, II-203, II-225, II-233, II-295, I'-6, I'-20, 1' 33, I'-46, 1' 58, 1' 66, 1' 79, I'-123, I'-124, I'-126, I'-130, I'-133, I'-136, I'-137, I'-139, I'-141, I'-142, I'-143, I'-145, I'-147, I'-151, and I'-153, or a pharmaceutically acceptable salt thereof.

3. The pharmaceutical preparation according to claim 2,
wherein (A) is a compound selected from the group consisting of compounds I-253, I-275, II-103, II-121, II-203, II-225, and II-233, or a pharmaceutically acceptable salt thereof.

4. The pharmaceutical preparation according to claim 2,
wherein (A) is a compound selected from the group consisting of compounds I'-6, I'-66, I'-124, I'-137, I'-142, I'-147, and I'-151, or a pharmaceutically acceptable salt thereof.

5. The pharmaceutical preparation according to any one of claims 1 to 4,
wherein (B) is at least one selected from orlistat, cetilistat, phentermine, mazindol, benzphetamine, amfepramone, methamphetamine, phentermine hydrochloride/topiramate, naltrexone hydrochloride/bupropion hydrochloride, liraglutide, semaglutide, setmelanotide, metreleptin, topiramate, naltrexone, bupropion, acarbose, voglibose, miglitol, ipragliflozin, dapagliflozin, remogliflozin, KGT-1075, luseogliflozin, tofogliflozin, canagliflozin, empagliflozin, ertugliflozin, bexagliflozin, enavogliflozin henagliflozin, janagliflozin, sotagliflozin, insulin aspart, insulin lispro, insulin glulisine, biosynthetic human neutral insulin, human insulin, biosynthetic human isophene insulin, human isophene insulin, intermediate insulin lispro, insulin detemir, insulin glargine, insulin degludec, glibenclamide, gliclazide, glimepiride, glipizide, gliquidone, nateglinide, mitiglinide calcium hydrate, repaglinide, metformin hydrochloride, buformin hydrochloride, pioglitazone hydrochloride, rosiglitazone, lobeglitazone, sitagliptin phosphate, vildagliptin, alogliptin benzoate, linagliptin, teneligliptin hydrobromide, anagliptin, saxagliptin, trelagliptin succinate, omarigliptin, gemigliptin, evogliptin, lixisenatide, exenatide, dulaglutide, tirzepatide, imegurimin, sitagliptin phosphate/ipragliflozin, pioglitazone hydrochloride/metformin, pioglitazone hydrochloride/glimepiride, teneligliptin hydrobromide/canagliflozin, alogliptin benzoate/pioglitazone hydrochloride, alogliptin benzoate/metformin hydrochloride, vildagliptin/metformin hydrochloride, mitiglinide calcium/voglibose, pravastatin, simvastatin, fluvastatin, atorvastatin, pitavastatin, rosuvastatin, lovastatin, clinofibrate, clofibrate, bezafibrate, fenofibrate, ciprofibrate, pemafibrate, gemfibrozil, colestimide, colestyramine, colesevelam hydrochloride, colestipol, ezetimibe, blobcall, nicomol, tocopherol nicotinic acid ester, niceritrol, ethyl icosapentate, ω -3 fatty acid ethyl, evolocumab, alirocumab, nifedipine, amlodipine, efonidipine, cilnidipine, nicardipine, nisoldipine, nitrendipine, nilvadipine, barnidipine, felodipine, benidipine, manidipine, azelnidipine, alanidipine, diltiazem, trichlormethiazide, benzylhydrochlorothiazide, hydrochlorothiazide, macrone, indapamide, tripamide, mefluside, furosemide, triamterene, spironolactone, eplerenone, tolvaptan, torasemide, hydrochlorothiazide, bumetanide, chlortalidone, isosorbide, metolazone,losartan, candesartan, valsartan, telmisartan, olmesartan, irbesartan, azilsartan, captopril, enalapril, alacepril, delapril, cilazapril, lisinopril, benazepril, imidapril, temocapril, quinapril, trandolapril, perindopril erbumine, urapidil, terazosin, prazosin, doxazosin, bunazosin, atenolol, bisoprolol, metoprolol, acebutolol, celiprolol, propranolol, nadolol, nipradilol, carteolol, pindolol, nebivolol, carvedilol, labetalol, sotalol, landiolol, arotinolol, amosulalol, arotinolol, carvedilol, labetalol, bevantolol, clonidine, guanabenz, methyldopa, reserpine, hydralazine, nitroprusside, aliskiren, kallidinogenase, alprostadil alfadex, dihydroergotoxine, doxazosin, urapidil, hydralazine, prazosin, moxonidine, guanfacine, rilmenidine, amlodipine/atrovastatin, losartan/hydrochlorothiazide, valsartan/hydrochlorothiazide, candesartan/hydrochlorothiazide, telmisartan/hydrochlorothiazide, irbesartan/trichlorothiazide, valsartan/amlodipine, olmesartan/azelnidipine, candesartan/amlodipine, telmisartan/amlodipine, irbesartan/amlodipine, valsartan/cilnidipine, azilsartan/amlodipine, tirzepatide, SCO-094, HM12525A, BI-456906, DD01, NN-9423, LY-3437943, danuglipron, PF07081532, LY-3502970, RGT-075, efpeglenatide, exenatide, Noiiglutide, GMA105, HM-15136, noliglycopeptide, LV-101, PYY-1562, PYY-1875, cotadutide/AM833, cotadutide, LY3305677, pegapamodutide, NGM395, YH34160, CT-388, CT-868, SCO-267, SCO-792, diazoxide, tesofensine, namodenoson, ERX1000, AMG133, ASC41, Xlal, HDV Biotin, EMP16, metoprolol/tesofensine, RZL-012, CB4211, BI1356225, AMG171, NO-13065, bardoxolone methyl, HSG4112, YHC2129, YHC2134, KTX-0200, obeticholic acid, GS-9674, LJN452, EDP-305, EYP-001, resmetirom, VK-2809, cenicriviroc, saroglitazar, lanifibranor, selonsertib, PF-06835919, pegbelfermin, efrugxifermin, aldafermin, aramcol, MK-3655, MSDC-0602K, belapectin, GS-0976, PF-05221304, ervogastat, ION-224, AXA-1125, HU-6, MET-409, MET-642, TERN-101, TERN-501, LPCN-1144, denifanstat, fruxifermin, leronlimab, pegozafermin, rencofilstat, retatrutide, tipelukast, S-237648, and S-723595.

6. The pharmaceutical preparation according to any one of claims 1 to 4, wherein (B) is at least one selected from GLP-1 agonists, GLP-1 analogs, NPY Y5 receptor antagonists, and ACC2 inhibitors.

7. The pharmaceutical preparation according to claim 6, wherein (B) is at least one selected from liraglutide, semaglutide, tirzepatide, BI-456906, danuglipron, PF07081532, LY-3502970, RGT-075, S-237648, S-723595, and pharmaceutically acceptable salts thereof.

8. The pharmaceutical preparation according to any one of claims 1 to 7, wherein (A) and (B) are administered in combination.

9. The pharmaceutical preparation according to any one of claims 1 to 7 which is a combination drug.

10. The pharmaceutical preparation according to any one of claims 1 to 9 for prevention and/or treatment of at least one selected from obesity, metabolic syndrome, hyperlipidemia, hypertriglyceridemia, hyper-VLDL-triglyceridemia, hyperfattyacidemia, diabetes mellitus, and arteriosclerosis.

11. An effect enhancer for (B) according to claim 1, comprising (A) according to claim 1.

12. An effect enhancer for (A) according to claim 1, comprising (B) according to claim 1.

13. A pharmaceutical preparation for administration in combination with (B) of claim 1, comprising (A) according to claim 1 as an active ingredient.

14. A pharmaceutical preparation for administration in combination with (A) according to claim 1, comprising (B) according to claim 1 as an active ingredient.
